(19) Europäisches Patentamt
European Patent Office
Office européen des brevets

(11) EP 3 138 540 A1

(12) EUROPEAN PATENT APPLICATION

(43) Date of publication:
08.03.2017 Bulletin 2017/10

(51) Int Cl.:
*A61F 5/01* (2006.01)

(21) Application number: 16189787.1

(22) Date of filing: 13.03.2009

(84) Designated Contracting States:
AT BE BG CH CY CZ DE DK EE ES FI FR GB GR
HR HU IE IS IT LI LT LU LV MC MK MT NL NO PL
PT RO SE SI SK TR

(30) Priority: 26.11.2008 US 324185

(62) Document number(s) of the earlier application(s) in
accordance with Art. 76 EPC:
09789515.5 / 2 381 902

(71) Applicant: Toad Corporation
Incline Village, NV 89451 (US)

(72) Inventors:
• FRANKE, Hans, G.
Incline Village, CA 89451 (US)

• SALINGER, David
Olympic Valley, CA 96146 (US)
• DODD, Jeff
Truckee, CA 96161 (US)

(74) Representative: Lawrence, John
Barker Brettell LLP
100 Hagley Road
Edgbaston
Birmingham B16 8QQ (GB)

Remarks:
This application was filed on 20-09-2016 as a
divisional application to the application mentioned
under INID code 62.

(54) **WEIGHT-BEARING LOWER EXTREMITY BRACE**

(57) The present disclosure relates, according to some embodiments to a system for redistributing weight away from a subject's foot or a method for facilitating ambulation of a subject, the system comprising: a platform (106; 306; 606; 706);

at least one vertical support (108, 308, 608, 708) fixed to the platform (106; 306; 606; 706) and extending upwardly from the platform; and

at least one cuff (110,310; 510; 610; 710) (a) configured to surround and releasably grip at least a portion of the subject's leg other than the foot and (b) mounted to the at least one vertical support (108, 308, 608, 708) at a vertical position along the at least one vertical support sufficient to suspend the subject's foot in a non-weight-bearing position above the platform (106; 306; 606; 706) during ambulation,

wherein the platform (106; 306; 606; 706), the at least one vertical support (108, 308, 608, 708), and the at least one cuff (110,310; 510; 610; 710) together are configured to bear at least the subject's full weight; and

wherein the at least one vertical support (108, 308, 608, 708) extends distal to the subject's foot.

FIG. 6B

**Description**

FIELD OF THE DISCLOSURE

The present disclosure relates, according to some embodiments, to methods, devices, and systems for ambulation of a subject having an impaired lower extremity.

BACKGROUND OF THE DISCLOSURE

Subjects unable to support their own weight due to a lower extremity impairment may resort to crutches or wheel chairs to move about. However, crutches and wheel chairs may be undesirable because of the limitations they impose on a subject's ability to use their hands. In addition, crutches and/or wheel chairs may incompletely restore mobility, and/or may be untenable in an older person due to decreased upper body strength and/or poor balance in the case of crutches.

SUMMARY

Accordingly, a need has arisen for improved ambulatory devices for subjects with impairments of the lower extremities. The present disclosure relates, according to some embodiments, to methods, devices, and systems for ambulation of a subject having an impaired lower extremity. For example, a device may comprise a lower extremity brace configured to bear a subject's weight and/or transfer the load to unimpaired organs and/or tissues.

According to some embodiments, a device and/or system (*e.g.*, redistributing weight away from a subject's foot) may comprise (a) a platform, (b) at least one vertical support fixed to the platform and extending upwardly from the platform, and (c) at least one cuff (i) configured to surround and releasably grip at least a portion of a subject's leg other than the foot and (ii) mounted (*e.g.*, fixedly, adjustably) to the at least one vertical support at a vertical position along the at least one vertical support sufficient to suspend a subject's foot in a non-weight-bearing position above the platform during ambulation, wherein the platform, the at least one vertical support, and the at least one cuff together are configured to bear at least the subject's full weight. A vertical support may comprise, in some embodiments, a strut positioned relative to the subject's leg on which the system is worn opposite the subject's sagittal plane or opposite the subject's coronal plane. For example, a strut may be positioned generally behind the subject's leg on which the system is worn. In some embodiments, a strut positioned generally behind the subject's leg on which the system is worn may have an S-curve profile. A vertical support may comprise, for example, a strut configured to extend along the outside of the leg on which the system is worn. In some embodiments, a vertical support may extend distal to the subject's foot (*e.g.*, extend from about the tibial plateau to beyond the bottom of the subject's foot). In some embodiments, a cuff may comprise an anterior shell, an opposing posterior shell, and at least one tension adjustment fastener configured to releasably connect the anterior shell and the posterior shell, wherein the anterior shell and the posterior shell are configured to combine to surround the circumference of the subject's leg. A cuff may comprise at least one collar (*e.g.*, adjustably mounted to the at least one vertical support), according to some embodiments. A collar may have, in some embodiments, opposing ends spaced apart and a tension adjustment fastener corresponding to each collar, each tension adjustment fastener configured to releasably connect the opposing ends of the corresponding collar, wherein each collar and corresponding tension adjustment fastener are together configured to surround the circumference of the subject's leg.

According to some embodiments, a cuff may further comprise at least one pad shell fixed to the at least one collar, and at least one pad fixed to the at least one pad shell. A pad shell may comprise a moldable plastic selected from the group consisting of a thermoplastic, a thermosetting plastic, and combinations thereof, in some embodiments. A pad may comprise, according to some embodiments, a material selected from the group consisting of polyurethane, polyethylene, neoprene, ethylene vinyl acetate, foam, silicone, rubber, and combinations thereof. In some embodiments, a cuff may include a second pad shell fixed to the at least one collar and a second pad fixed to the second pad shell. A cuff including two pads and pad shells may be arranged such that the first pad comprises an anterior pad, the first pad shell comprises an anterior pad shell, the second pad comprises a posterior pad, and the second pad shell comprises a posterior pad shell, according to some embodiments. A cuff may be configured to extend from about the gastrocnemius/solius muscles to about the tibial plateau, according to some embodiments. A device and/or system (*e.g.*, for redistributing weight away from a subject's foot) may comprise, in some embodiments, an outsole fixed to the platform and configured to contact the ground during ambulation. In some embodiments, an outsole may comprise a flange extending, for example, vertically along at least a portion of the back of the vertical support.

A shell (*e.g.*, anterior shell), according to some embodiments, may comprise at least one fastener guide positioned across the anterior surface of the anterior shell and configured to receive at least a portion of the at least one tension adjustment fastener. In some embodiments, a shell (*e.g.*, anterior shell, posterior shell) may comprise a moldable plastic selected from the group consisting of a thermoplastic, a thermosetting plastic, and combinations thereof. A shell (*e.g.,* anterior shell, posterior shell) may be mounted (*e.g.,* fixedly, adjustably) to the at least one vertical support in some embodiments. A shell (*e.g.*, anterior shell, posterior shell), according to some embodiments, may include an attached pad. For example, a cuff may include one or more pads fixed to (*e.g.,* lining) at least a portion of the surface(s) proximal to the subject's skin. Each cuff and/or each shell may include a pad. Each pad may independently comprise a material selected from the group consisting of polyurethane, polyethylene, neoprene, ethylene vinyl acetate, foam, silicone, rubber, and combinations thereof. In some embodiments, a cuff may comprise one or more bladders fixed to (*e.g.,* lining) at least a portion of the surface(s) proximal to the subject's skin. Each cuff and/or each shell may include a bladder. Each bladder may be filled (*e.g.,* at least partially filled, completely filled) with a fill material. A fill material may comprise a foam. A fill material may provide, for example, a cushioned and/or custom fit.

According to some embodiments, a device and/or system (*e.g.*, for redistributing weight away from a subject's foot) may be configured to secure a subject's leg in a bent (*e.g.*, slightly bent) position. A vertical support may comprise, for example, a vertical rod (*e.g.,* extending distal to the subject's foot). A lateral bar may be interposed between the at least one cuff and the vertical rod, in some embodiments. A device and/or system (*e.g.*, for redistributing weight away from a subject's foot) may comprise, according to some embodiments, a second cuff, the second cuff (a) configured to surround and releasably grip at least a second portion of a subject's leg other than the foot and (b) mounted (*e.g.*, fixedly, adjustably) to the at least one vertical support at a vertical position along the at least one vertical support sufficient to suspend a subject's foot in a non-weight-bearing position above the platform during ambulation.

In some embodiments, a device and/or system (*e.g.*, for redistributing weight away from a subject's foot) may comprise further (d) a hinge attached to the upper end of the vertical support, (e) a second vertical support rotatably attached to the hinge, and (f) a second cuff, the second cuff (i) configured to surround and releasably grip at least a second portion of a subject's leg other than the foot and (ii) mounted (*e.g.*, fixedly, adjustably to the second vertical support at a vertical position along the second vertical support sufficient to cooperate with the first cuff to suspend a subject's foot in a non-weight-bearing position above the platform during ambulation. A second cuff may be configured to extend from about the proximal patella to about the upper quadriceps. According to some embodiments, a device and/or system (*e.g.*, for redistributing weight away from a subject's foot) may comprise a mated shoe comprising a lift configured to vertically align the suspended foot and the other foot. In some embodiments, a strut may be configured to be contiguous with its adjacent platform.

The present disclosure relates, in some embodiments, to methods for facilitating ambulation of a subject having a leg with an impaired lower extremity. A method may comprise, for example, suspending at least a portion of the impaired lower extremity in a non-weight bearing position using a load redistribution system and redistributing the weight to one or more unimpaired regions of the leg using the load redistribution system. Redistributing the weight to one or more unimpaired regions of the leg may comprise, in some embodiments, redistributing the weight to one or more lateral surfaces (*e.g.*, unimpaired lateral surfaces) of the leg. According to some embodiments, a method may comprise adjusting the pressure applied to at least one lateral surface (*e.g.*, by substituting with a different contact surface area). A method (*e.g.*, for facilitating ambulation of a subject having a leg with an impaired lower extremity) may comprise, for example, contacting the leg with a load redistribution system to suspend at least the impaired lower extremity in a non-weight bearing position and redistributing at least the weight of the subject during ambulation to one or more unimpaired regions of the leg. A load redistribution system may include, in some embodiments, a platform, at least one vertical support fixed to the platform and extending upwardly from the platform, and at least one cuff mounted (*e.g.*, fixedly, adjustably) to the at least one strut at a position sufficient to suspend the at least a portion of the impaired lower extremity in a non-weight-bearing position above the platform during ambulation, wherein the platform, the at least one strut, and the at least one cuff are configured to (a) bear at least the subject's full weight and (b) distribute the weight born to at least a portion of the subject's leg other than the subject's foot.

In some embodiments, a device and/or system (*e.g.*, for redistributing weight away from a subject's foot) may comprise a means for suspending a subject's foot in a non-weight-bearing position and a means for redistributing a subject's weight to at least one surface on the subject's leg.

BRIEF DESCRIPTION OF THE DRAWINGS

Some embodiments of the disclosure may be understood by referring, in part, to the present disclosure and the accompanying drawings, wherein:

FIGURE 1A illustrates a generally isometric view of a device for ambulation of a subject having an impaired lower extremity according to a specific example embodiment of the disclosure;
FIGURE 1B illustrates a left profile view of the device for ambulation of a subject shown in FIGURE 1A according to a specific example embodiment of the disclosure;
FIGURE 1C illustrates a right profile view of the device for ambulation of a subject shown in FIGURE 1A according to a specific example embodiment of the disclosure;

FIGURE 1D illustrates a front view of the device for ambulation of a subject shown in FIGURE 1A according to a specific example embodiment of the disclosure;

FIGURE 2A illustrates a generally isometric view of a device for ambulation of a subject having an impaired lower extremity according to a specific example embodiment of the disclosure;

FIGURE 2B illustrates a generally isometric view of a device for ambulation of a subject having an impaired lower extremity according to a specific example embodiment of the disclosure;

FIGURE 3A illustrates a generally isometric view of a device for ambulation of a subject having an impaired lower extremity according to a specific example embodiment of the disclosure;

FIGURE 3B illustrates a left profile view of the device for ambulation of a subject shown in FIGURE 3A according to a specific example embodiment of the disclosure;

FIGURE 3C illustrates a right profile view of the device for ambulation of a subject shown in FIGURE 3A according to a specific example embodiment of the disclosure;

FIGURE 3D illustrates a front view of the device for ambulation of a subject shown in FIGURE 3A according to a specific example embodiment of the disclosure;

FIGURE 4 illustrates a profile view of a device for ambulation of a subject having an impaired lower extremity according to a specific example embodiment of the disclosure;

FIGURE 5A illustrates a front view of a device for ambulation of a subject having an impaired lower extremity according to a specific example embodiment of the disclosure;

FIGURE 5B illustrates a generally isometric view of a cuff according to a specific example embodiment of the disclosure;

FIGURE 6A illustrates a generally isometric view of a device for ambulation of a subject having an impaired lower extremity according to a specific example embodiment of the disclosure;

FIGURE 6B illustrates a generally isometric view of the device for ambulation of a subject shown in FIGURE 6A according to a specific example embodiment of the disclosure.

FIGURE 6C illustrates a generally isometric view of the device for ambulation of a subject shown in FIGURE 6A according to a specific example embodiment of the disclosure;

FIGURE 6D illustrates a generally isometric view of the device for ambulation of a subject shown in FIGURE 6A according to a specific example embodiment of the disclosure;

FIGURE 6E illustrates a generally isometric view of the device for ambulation of a subject shown in FIGURE 6A according to a specific example embodiment of the disclosure;

FIGURE 6F illustrates a right profile view of the device for ambulation of a subject shown in FIGURE 6A according to a specific example embodiment of the disclosure;

FIGURE 6G illustrates a left profile view of the device for ambulation of a subject shown in FIGURE 6A according to a specific example embodiment of the disclosure;

FIGURE 6H illustrates a rear view of the device for ambulation of a subject shown in FIGURE 6A according to a specific example embodiment of the disclosure;

FIGURE 7A illustrates a generally isometric view of a device for ambulation of a subject having an impaired lower extremity according to a specific example embodiment of the disclosure;

FIGURE 7B illustrates a generally isometric view of the device for ambulation of a subject shown in FIGURE 7A according to a specific example embodiment of the disclosure;

FIGURE 7C illustrates a left profile view of the device for ambulation of a subject shown in FIGURE 7A according to a specific example embodiment of the disclosure;

FIGURE 7D illustrates a left profile view of the device for ambulation of a subject shown in FIGURE 7A according to a specific example embodiment of the disclosure;

FIGURE 7E illustrates a left profile view of the device for ambulation of a subject shown in FIGURE 7A according to a specific example embodiment of the disclosure;

FIGURE 7F illustrates an exploded view of the device for ambulation of a subject shown in FIGURE 7A according to a specific example embodiment of the disclosure;

FIGURE 7G illustrates an exploded view of the device for ambulation of a subject shown in FIGURE 7A according to a specific example embodiment of the disclosure; and

FIGURE 7H illustrates a generally isometric view of the device for ambulation of a subject shown in FIGURE 7A according to a specific example embodiment of the disclosure.

DETAILED DESCRIPTION

The present disclosure relates, according to some embodiments, to methods, devices, and systems for facilitating ambulation (*e.g.*, walking, running, dancing, and the like) of a subject having an impaired lower extremity. For example, a system for facilitating ambulation of a subject may include a walking brace configured to suspend a subject's lower extremity (*e.g.*, foot) in a non-weight-bearing position. In some embodiments, a system may transfer the subject's weight to at least a portion of the subject's lower leg and/or to at least a portion of the subject's upper leg. A weight-bearing portion of the lower leg may have a vertical span from about the gastrocnemius/solius muscles to about the tibial plateau. A weight-bearing portion of the upper leg may have a vertical span from just superior to the patella to about the proximal one third of the quadriceps femoris. A lower cuff may span up to the entire circumference of a subject's lower leg. An upper cuff may span up to the entire circumference of a subject's upper leg. When positioned upright, a subject's foot may dangle loosely, The subject's impaired lower extremity may have a wound dressing, a cast, a wrap, or other dressings or garments. With respect to a cast (and/or any other rigid and/or semi rigid material), care may be taken to ensure that the cast does not bear any weight since that load may be transmitted to a subject's foot. For example, a device may be adapted to accommodate a subject wearing a cast that covers the subject's ankle and extends up to about the mid-calf region (*e.g.*, by shortening the lower cuff by an amount sufficient to ensure that weight is not transferred from the brace to the cast). It may be desirable, in some embodiments, to include a covering and/or sling to protect and support a lower extremity. The choice of covering and/or sling may be influenced by the nature of the impairment and/or the nature of the dressing.

For example, it may be desirable to support and/or immobilize a subject's foot in a position that is generally perpendicular to the axis of the lower leg (*e.g.,* to prevent and/or minimize Achilles tendon contracture). For example, a sling attached (*e.g.*, by a slide or spring to avoid permitting the foot to bear weight) to at least one cuff and/or at least one strut may be used to secure a subject's foot in this position. Alternatively, a platform may be fitted with one or more pads on its upper surface with the pad positioned, for example, under the ball of the foot or over the entire platform. A foot pad, in some embodiments, may be scored or perforated into segments to allow a health care worker or a subject to customize the support as needed. For example, it may be desirable to minimize Achilles tendon contracture in a subject with an open sore *(e.g.,* diabetic foot ulcer) by providing foot support that does not contact the open sore. Segments of the pad may be selectively removed to avoid that contact.

According to some embodiments, an impairment of a lower extremity may include a foot impairment, an ankle impairment, and/or a knee impairment. An impairment may affect a subject's ability to bear weight on or near the effected region. For example, an impairment may exist where the pathology itself impedes the weight bearing capacity. An impairment may also exist where therapy, convalescence, and/or rehabilitation include relieving the region of weigh-bearing forces. Examples of lower extremity impairments may include, without limitation, a fractured bone, a broken bone, a sprain, an ulcer (*e.g.,* a diabetic ulcer), arthritis, a joint dislocation, a joint subluxation, a torn ligament (*e.g.*, a torn anterior cruciate ligament), a torn cartilage (*e.g.*, a torn meniscus), bursitis, tendonitis, an infection, gout, gangrene, plantar fasciitis, metabolic diseases, bone or cartilage diseases, neuropathic states and/or post-operative states.

In some embodiments, increased ambulation may reduce, eliminate, and/or prevent one or more conditions, including, for example, deep venous thrombosis, atrophy, pain (*e.g.,* lower back pain due to bed rest), and/or osteoporosis or other bone loss. Increasing a subject's ambulation without crutches may decrease the incidence of injuries associated with falling and/or upper body strain.

A load redistribution system, in some embodiments, may include a platform, at least one vertical strut fixed to the platform, and at least one cuff fixed to the at least one vertical strut. In some embodiments, one or more fasteners may be used to secure a load redistribution system to a subject's leg including, for example, belts, buckles (*e.g.*, ski boot buckles, ratcheting buckles), buttons, cams, carabineers, chains, cinches, clasps, D-rings, draw-strings, hooks, levers, locks, loops, slides, snaps, straps, and/or tensioners. These fasteners may be attached to the at least one vertical strut and/or the at least one cuff.

According to some embodiments, a load redistribution system may be configured to bear and/or redistribute a load (*e.g.*, a subject's weight). For example, a load redistribution system may be configured to transfer the load of s subject's weight away from the subject's foot and transfer it to the subject's lower and/or upper leg. A load redistribution system may comprise one or more struts positioned adjacent to a subject's leg (*e.g.,* in front of, behind, to the right, of and/or to the left of a subject's leg). For example, a single strut may be positioned on the left side of a left leg, on the right side of a right leg, or behind a left leg or right leg. In some embodiments, a strut may be positioned on the generally opposite side of a leg from a subject's sagittal plane or on the generally opposite side of a leg from a subject's coronal plane. In some embodiments, a strut positioned generally behind the subject's leg on which the system is worn may have an S-curve profile. For example, a strut may be configured to have a profile that resembles a letter "S" in that it has two opposing inflection points (*e.g.*, one anterior and one posterior). An S-curve profile may permit clearance for a subject's foot and/or a normal stride.

A strut may comprise, according to some embodiments, two pieces (*e.g.,* an upper piece and a lower piece) joined by a hinge. In some embodiments, a hinge may be configured to permit a limited range *(e.g.,* limited flexion and/or extension) and/or an unlimited range of rotational motion. According to some embodiments, a two-strut system may have a strut on each side of a subject's leg, with each strut having a single piece (*e.g.*, lower leg only) or two pieces connected by a hinge (*e.g.,* a full leg brace).

According to some embodiments, a load redistribution system may be configured to bear a load. A load may include, for example, a subject's weight, a portion of a subject's weight, and/or a multiple of subject's weight (*e.g.*, 1.1x, 1.2x, 1.5x, and/or 2x). It may be desirable for a load redistribution system to bear a more than a subject's weight, for example, where the subject may carry additional weight (*e.g.*, a backpack, a bag of groceries, a child, and/or the like) and/or may put the system under additional stress (*e.g.*, through sport, exercise, or the like). In some embodiments, a subject's lower extremity (*e.g.*, foot) with a load redistribution system in place bears little or no weight, even though weight is born by that leg (*e.g.,* while standing, exercising, working, and/or engaging in other activities).

A load redistribution system may include at least one cuff fixed to at least one strut in some embodiments. For example, a system may include a lower leg cuff and an upper leg cuff. A system may include a plurality of lower cuffs and/or a plurality of upper cuffs in some embodiments. For example, if a single cuff would contact and/or cover an injured portion of a lower leg, it may be desirable to instead use two or more lower cuffs configured to minimize and/or avoid contacting/covering the effected region. A cuff may be fixedly or adjustably mounted to a corresponding strut in some embodiments. For example, a strut may include a series of holes (*e.g.*, equidistantly spaced) configured to receive a corresponding pin (*e.g.*, spring-loaded) and/or screw attached to a cuff. A user may slide the cuff along the length of a strut and engage the screw and/or pin when a desired position is found, In some embodiments, a load redistribution system may include continuously variable adjustment system. A cuff may slide along the length of a strut with one or more set screws configured to fix the relative positions of each once a desirable position is found.

In some embodiments, a cuff may include a pad disposed to contact at least a portion of a subject's leg and an outer shell. An inner pad may comprise any desirable gel, foam, and/or other cushioning material(s). A custom fit cuff may facilitate, according to some embodiments, uniform distribution (*e.g.*, uniform, substantially uniform, relatively uniform) of a subject's weight over the surface area of the cuff An outer shell may comprise a moldable material.

In some embodiments, a cuff may be opened on at least one side to facilitate donning and/or removing the cuff (and attached load redistribution system). For example, a cuff may comprise two outer shells with one or more hinges arranged on one side to allow the cuff to open clamshell-style. Two or more outer shells may fit together without a hinge or other permanent connection according to some embodiments. Once donned, a cuff may be closed and/or secured using any type of fixed or adjustable tensioning system. For example, one or more collars, straps, cinches, guides, loops, hooks, hoops, buckles, and/or combinations thereof may be used. In some embodiments, a tensioning system may include hooks and/or loops (*e.g.*, VELCRO®).

According to some embodiments, a load redistribution system, when donned, will increase a subject's inseam. This may result in an undesirable difference between the inseam of the leg on which a load redistribution system is worn and the inseam of the free leg. Any difference may be offset, at least partially, by wearing a lift (*e.g.*, integrated and/or inserted in a shoe) or other apparatus on the free leg.

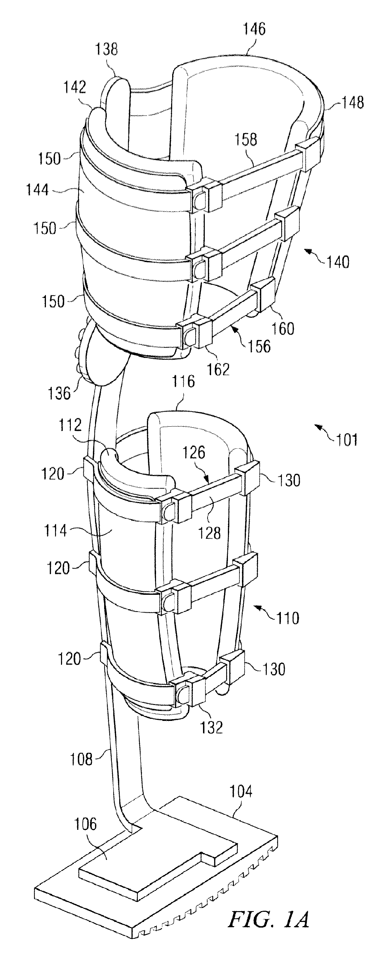

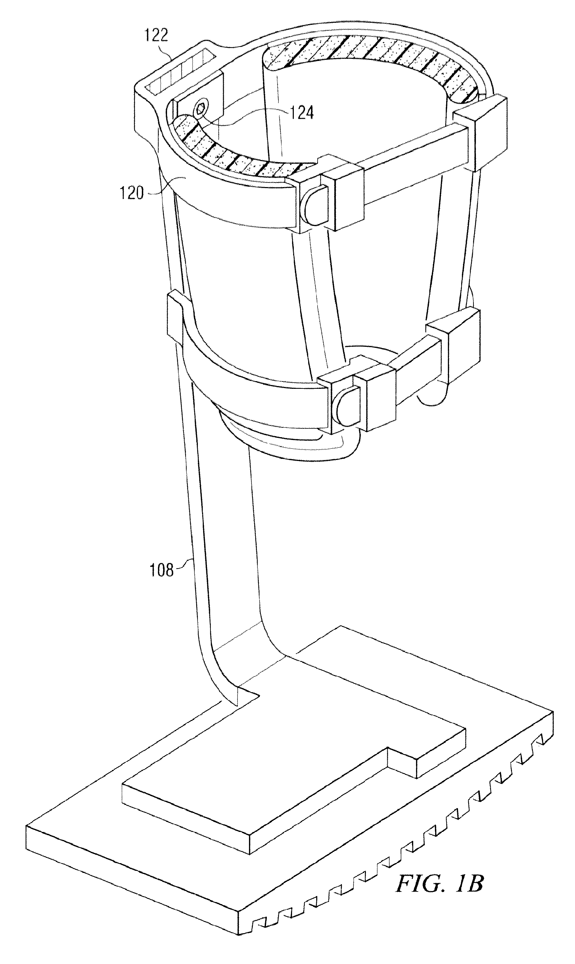

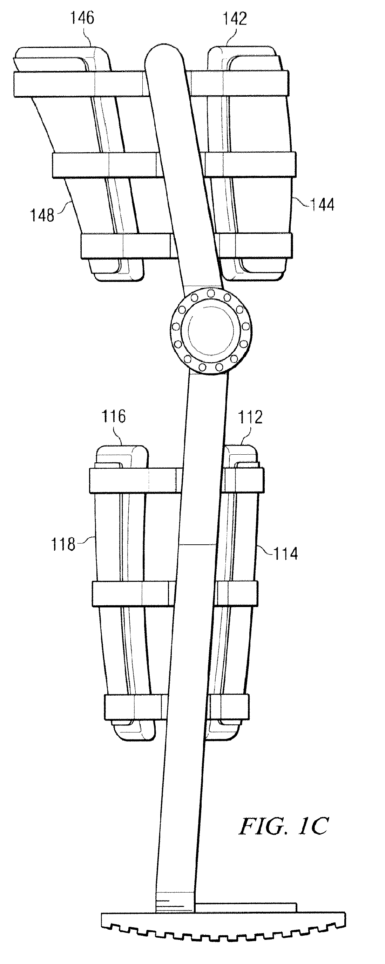

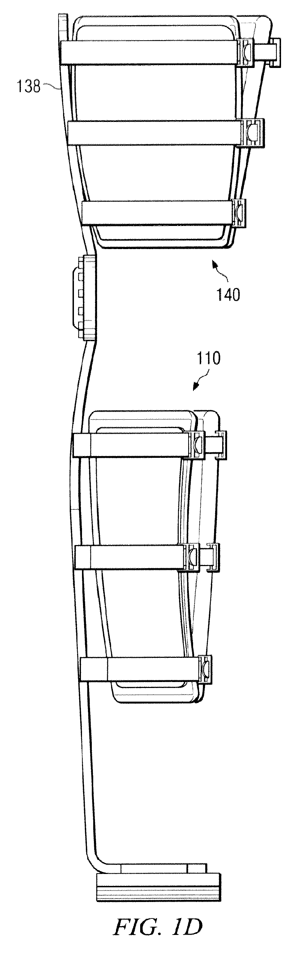

FIGURES 1A-1D illustrate an example of a load redistribution system **101** for ambulation of a subject having a lower extremity impairment, in accordance with embodiments of the present disclosure. As shown in FIGURES 1A-1D, load redistribution system **101** may include outsole **104,** platform **106,** lower strut **108**, lower cuff **110,** hinge **136,** upper strut **138**, and upper cuff **140.**

Outsole **104** may comprise a foot pad, the bottom of which may be rounded (*e.g.*, rocker outsole) to facilitate walking. The bottom may be ridged as pictured or may have another tread pattern to aid ambulation. Outsole **104** may comprise a hard rubber or other suitable material. Outsole **104** may be generally rectangular in shape as depicted. Other regular and/or irregular shapes may be suitable and/or desirable in some embodiments. Platform **106** may be contiguous with lower strut **108** as shown or may be a separate piece fixedly attached (*e.g.*, welded, bolted) to lower strut **108.** In some embodiments, outsole **104** and platform **106** may be adjustable (*e.g.*, fore and aft) relative to each other. The lower surface of platform **106** may be affixed to outsole **104** with any type of fastener and/or adhesive. Platform **106** may sit atop outsole **104** as shown. In some embodiments, platform **106** may be recessed within outsole **104,** for example, so that the upper surface of outsole **104** is flush with the upper surface of platform **106**. Platform **106** may have any regular or irregular shape. Platform **106** may be somewhat smaller than outsole **104** as depicted or may be sized to match the size of outsole **104**. In some embodiments, it may be desirable for platform **106** to be larger than outsole **104.** Platform **106** may comprise a rigid material suitable for bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof.

Lower strut **108** may extend vertically from platform **106** to hinge **136**. Upper strut **138** may extend vertically from hinge **136** to a position corresponding to or just below a subject's hip. Lower strut **108** may have a lengthwise axis that is approximately parallel to the lengthwise axis of a subject's leg (*e.g.*, lower leg). Upper strut **138** may have a lengthwise axis that is approximately parallel to the lengthwise axis of a subject's leg *(e.g.,* upper leg). Lower strut **108** and/or upper strut **138** may comprise one or more rigid materials capable of bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof.

As depicted, lower cuff **110** may comprise anterior pad **112,** anterior pad shell **114,** posterior pad **116,** posterior pad shell **118,** calf collar **120,** and tension adjustment fastener **126,** Pads **112** and/or **116** may independently comprise flexible, elastomeric, and/or resilient materials including, for example, polyurethane, polyethylene, neoprene, ethylene vinyl acetate, foam, silicone, rubber, and the like. Pad shells **114** and/or **118** may independently comprise a moldable plastic. In some embodiments, pad shells **114** and/or **118** may independently comprise steel, a steel alloy, aluminum, an aluminum alloy, titanium, a titanium alloy, carbon fiber, an aramid fiber, a para-aramid fiber (*e.g.*, KEVLAR), fiberglass, and combinations thereof. Pad shells **114** and/or **118** comprising a moldable material may be custom fit to a subject. A moldable plastic may include, for example, a thermoplastic (*e.g.*, capable of more than one cycle of melting, molding, and setting) and/or a thermosetting plastic (*e.g.*, capable of one cycle of melting, molding, and setting). Pads **112** and/or **116** may be adhered, welded, bonded, stitched, or otherwise fixed to pad shells **114** and/or **118**, respectively.

Pad shells **114** and/or **118** may be fixedly attached to calf collar **120**. Calf collar **120** may extend up to all the way around the circumference of a subject's lower leg. Calf collar **120** may include sleeve **122** positioned, for example, at or near its midpoint. Sleeve **122** may be configured to receive strut **108**. Sleeve **122** may have a cavity, the longitudinal axis of which is about perpendicular to the lengthwise axis of strut **108**. Sleeve **122** may be configured to allow collar **120** to slide along the length of strut **108**. Sleeve **122** may include set screw **124** positioned to adjustably contact strut **108**. Each calf collar may be configured to include a sleeve and each sleeve may include a set screw. In use, set screw **124** may be loosened to permit collars **120** to slide along the length of strut **108**. Once a desirable, position is found, set screw **124** may be tightened to fix the position of collar **120** on strut **108**. Set screw **124** may be positioned on the inside of collar **120** and directed outwardly toward strut **108** as shown. In some embodiments, set screw **124** may be positioned on the outside of collar **120** and directed inwardly toward strut **108**. As depicted, lower cuff **110** may include three calf collars **120**. In some embodiments, more or fewer collars may be desired and/or required.

Tension adjustment fastener **126** may include strap **128,** cinch **130,** and anchor **132**. Strap **128** maybe fixed to one end of calf collar **120** via anchor **132**. Cinch **130** may be fixed to the other end of collar **120**. Cinch **130** may be configured to receive and releasably grip strap **128**. In use, strap **128** may be threaded through cinch **130** and pulled tight to apply a desired amount of pressure on the subject's lower leg. Cinch **130** may then be closed to fix strap **128** in its position. As depicted, the loose end of strap **120** may be tucked into anchor **132**.

Hinge **136** may link lower strut **108** and upper strut **138** and permit rotation of lower strut **108** and upper strut **138** relative to each other. When system **101** is in position on a subject's leg, the hinge axis of hinge **136** may be parallel or substantially parallel to the hinge axis of the subject's knee in good health. It may be desirable, in some embodiments, to configure hinge **136** to have a limited and/or selectable degree of rotation (*e.g.*, flexion and/or extension).

As depicted, upper cuff **140** may comprise anterior pad **142,** anterior pad shell **144,** posterior pad **146,** posterior pad shell **148,** thigh collar **150,** and tension adjustment fastener **156**. Pads **142** and/or **146** may independently comprise flexible, elastomeric, and/or resilient materials including, for example, polyurethane, polyethylene, neoprene, ethylene vinyl acetate, foam, silicone, rubber, and the like.

In some embodiments, pad shells **144** and/or **148** may independently comprise a moldable plastic, steel, a steel alloy, aluminum, an aluminum alloy, titanium, a titanium alloy, carbon fiber, an aramid fiber, a para-aramid fiber (*e.g.*, KEVLAR), fiberglass, and combinations thereof. Pad shells **144** and/or **148** comprising a moldable material may be custom fit to a subject. A moldable plastic may include, for example, a thermoplastic (*e.g.*, capable of more than one cycle of melting, molding, and setting) and/or a thermosetting plastic (*e.g.*, capable of one cycle of melting, moulding, and setting). Pads **142** and/or **146** may be adhered, welded, bonded, stitched, or otherwise fixed to pad shells **144** and/or **148,** respectively.

Pad shells **144** and/or **148** may be fixedly attached to thigh collar **150**. Thigh collar **150** may extend up to all the way around the circumference of a subject's upper leg. Thigh collar **150** may include sleeve **152** positioned, for example, at or near its midpoint. Sleeve **152** may be configured to receive strut **138**. Sleeve **152** may have a cavity, the longitudinal axis of which is about perpendicular to the lengthwise axis of strut **138**. Sleeve **152** may be configured to allow collar **150** to slide along the length of strut **138**. Sleeve **152** may include set screw **154** positioned to adjustably contact strut **138**. In use, set screw **154** may be loosened to permit collar **150** to slide along the length of strut **138**. Once a desirable position is found, set screw **154** may be tightened to fix the position of collar **150** on strut **138**. Set screw **154** may be positioned on the inside of collar **150** and directed outwardly toward strut **138** as shown. In some embodiments, set screw **154** may be positioned on the outside of collar **150** and directed inwardly toward strut **138**. As depicted, upper cuff **140** may include three thigh collars **150**. In some embodiments, more or fewer collars may be desired and/or required.

Tension adjustment fastener **156** may include strap **158,** cinch **160,** and anchor **162**. Strap **158** maybe fixed to one end of thigh collar **150** via anchor **162**. Cinch **160** may be fixed to the other end of collar **150**. Cinch **160** may be configured to receive and releasably grip strap **158**. In use, strap **158** may be threaded through cinch **160** and pulled tight to apply a desired amount of pressure on the subject's upper leg. Cinch **160** may then be closed to fix strap **158** in its position. As depicted, the loose end of strap **150** may be tucked into anchor **162**.

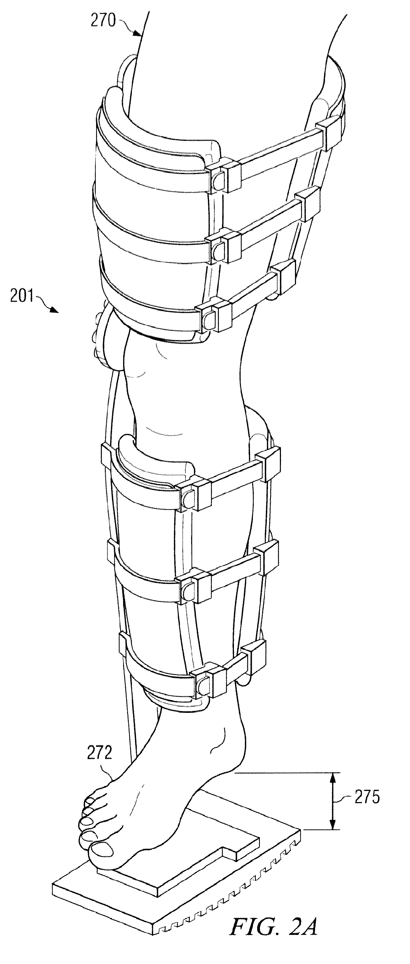

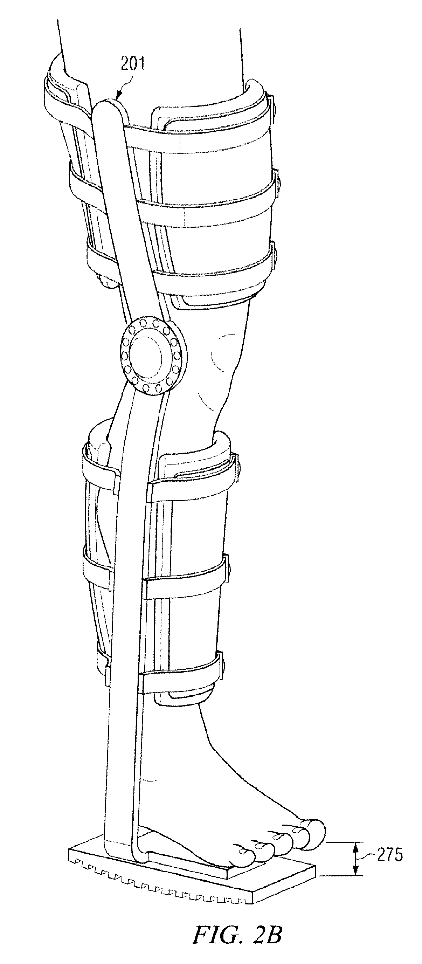

FIGURES 2A-2B illustrate an example load redistribution system **201** for ambulation of a subject having a lower extremity impairment, in accordance with embodiments of the present disclosure. As shown in FIGURES 2A-2B, system **201** may be configured (*e.g.*, formed and/or adjusted) to include gap **275** between a subject's foot **272** and the higher of outsole **204** and platform **206**. Gap **275** relieves subject's foot **272** and/or other lower extremities from bearing weight by diverting the load elsewhere. In some embodiments, gap **275** may be any suitable distance that avoids (*e.g.*, minimizes, eliminates) loading foot **272** and/or other lower extremities. Gap **275** may depend, in part, upon the size and weight of the subject user. For example, a subject with a long foot may be inclined and/or compelled to support some weight on foot **272** at some points in a walking cycle (*e,g.,* when foot **272** is at it's most rearward position). It may be desirable in such situations to adjust gap **275** to be larger. A subject, for example, a smaller subject, may desire and/or require a smaller gap **675,** for example, to reduce or minimize the difference in length between the effected leg with system **602** and a healthy leg. In some embodiments, gap **275** may be from about 1 cm to about 5 cm, from about I cm to about 15 cm, from about 1 cm to about 10 cm, from about 1 cm to about 20 cm, from about 2 cm to about 5 cm, from about 2 cm to about 10 cm, from about 2 cm to about 15 cm, and/or from about 2 cm to about 20 cm. Gap **275** may be less than about 1 cm or more than 20 cm in some embodiments.

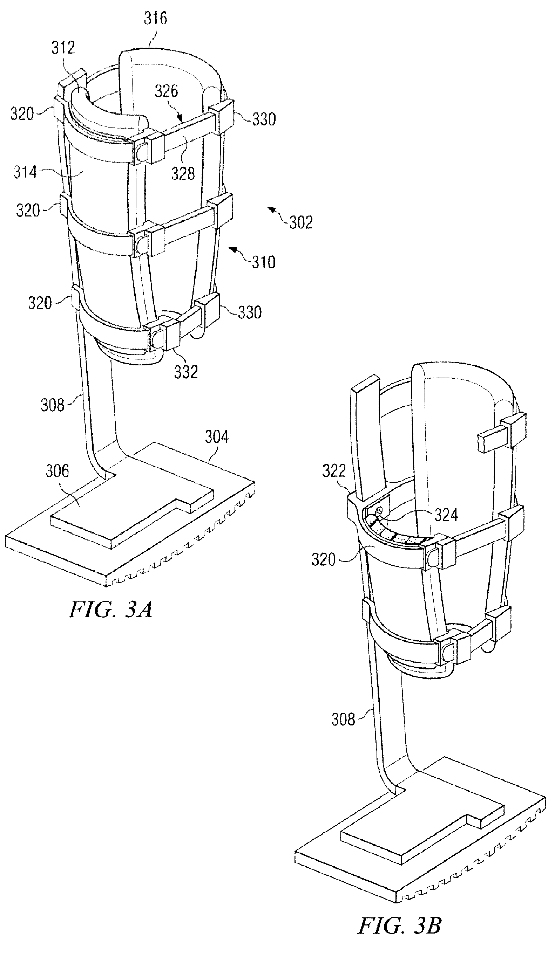

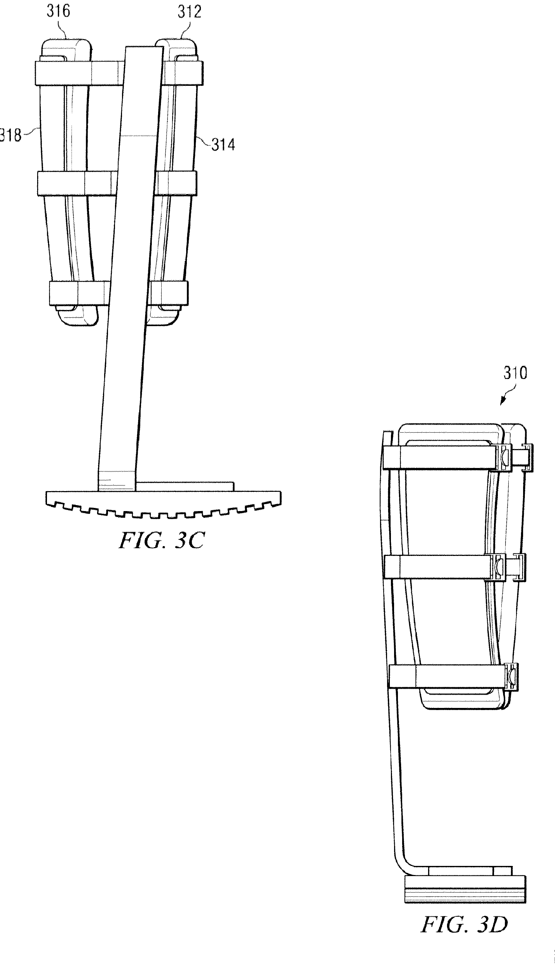

FIGURES 3A-3D illustrate an example of a load redistribution system **302** for ambulation of a subject having a lower extremity impairment, in accordance with embodiments of the present disclosure. As shown in FIGURES 3A-3D, load redistribution system **302** may include outsole **304**, platform **306,** lower strut **308,** and lower cuff **310.**

Outsole **304** may comprise a foot pad, the bottom of which may be rounded (*e.g.*, rocker outsole) to facilitate walking. The bottom may be ridged as pictured or may have another tread pattern to aid ambulation. Outsole **304** may comprise a hard rubber or other suitable material. Outsole **304** may be generally rectangular in shape as depicted. Other regular and/or irregular shapes may be suitable and/or desirable in some embodiments. Platform **306** may be contiguous with strut **308** as shown or may be a separate piece fixedly attached to strut **308**. The lower surface of platform **306** may be affixed to outsole **304** with any type of fastener and/or adhesive. Platform **306** may sit atop outsole **304** as shown. In some embodiments, platform **306** may be recessed within outsole **304,** for example, so that the upper surface of outsole **304** is flush with the upper surfaces of platform **306**. Platform **306** may have any regular or irregular shape. Platform **306** may be somewhat smaller than outsole **304** as depicted or may be sized to match the size of outsole **304**. In some embodiments, it may be desirable for platform **306** to be larger than outsole **304**. Platform **306** may comprise a rigid material suitable for bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof.

Strut **308** may extend vertically from platform **306** to hinge to a position corresponding to or just below a subject's tibia. Strut **308** may have a lengthwise axis that is approximately parallel to the lengthwise axis of a subject's leg (*e.g.,* lower leg). Strut **308** may comprise one or more rigid materials capable of bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof.

As depicted, lower cuff **310** may comprise anterior pad **312,** anterior pad shell **314,** posterior pad **316,** posterior pad shell **318,** calf collar **320,** and tension adjustment fastener **326**. Pads **312** and/or **316** may independently comprise flexible, elastomeric, and/or resilient materials including, for example, polyurethane, polyethylene, neoprene, ethylene vinyl acetate, foam, silicone, rubber, and the like. In some embodiments, pad shells **314** and/or **318** may independently comprise a moldable plastic, steel, a steel alloy, aluminum, an aluminum alloy, titanium, a titanium alloy, carbon fiber, an aramid fiber, a para-aramid fiber (*e.g.*, KEVLAR), fiberglass, and combinations thereof. Pad shells **314** and/or **318** comprising a moldable material may be custom fit to a subject. A moldable plastic may include, for example, a thermoplastic (*e.g.*, capable of more than one cycle of melting, molding, and setting) and/or a thermosetting plastic (*e.g.*, capable of one cycle of melting, molding, and setting). Pads **312** and/or **316** may be adhered, welded, bonded, stitched, or otherwise fixed to pad shells **314** and/or **318,** respectively.

Pad shells **314** and/or **318** may be fixedly attached to calf collar **320**. Calf collar **320** may extend up to all the way around the circumference of a subject's lower leg. Calf collar **320** may include sleeve **322** positioned, for example, at or near its midpoint. Sleeve **322** may be configured to receive strut **308**. Sleeve **322** may have a cavity, the longitudinal axis of which is about perpendicular to the lengthwise axis of strut **308**. Sleeve **322** may be configured to allow collar **320** to slide along the length of strut **308**. Sleeve **322** may include set screw **324** positioned to adjustably contact strut **308**. Each calf collar may be configured to include a sleeve and each sleeve may include a set screw. In use, set screw **324** may be loosened to permit collar **320** to slide along the length of strut **308**. Once a desirable position is found, set screw **324** may be tightened to fix the position of collar **320** on strut **308**. Set screw **324** may be positioned on the inside of collar **320** and directed outwardly toward strut **308** as shown. In some embodiments, set screw **324** may be positioned on the outside of collar **320** and directed inwardly toward strut **308**. As depicted, lower cuff **310** may include three calf collars **320**. In some embodiments, more or fewer collars may be desired and/or required.

Tension adjustment fastener **326** may include strap **328,** cinch **330,** and anchor **332**. Strap **328** maybe fixed to one end of calf collar **320** via anchor **332**. Cinch **330** may be fixed to the other end of collar **320**. Cinch **330** may be configured to receive and releasably grip strap **328**. In use, strap **328** may be threaded through cinch **330** and pulled tight to apply a desired amount of pressure on the subject's lower leg. Cinch **330** may then be closed to fix strap **328** in its position. As depicted, the loose end of strap **320** may be tucked into anchor **332**.

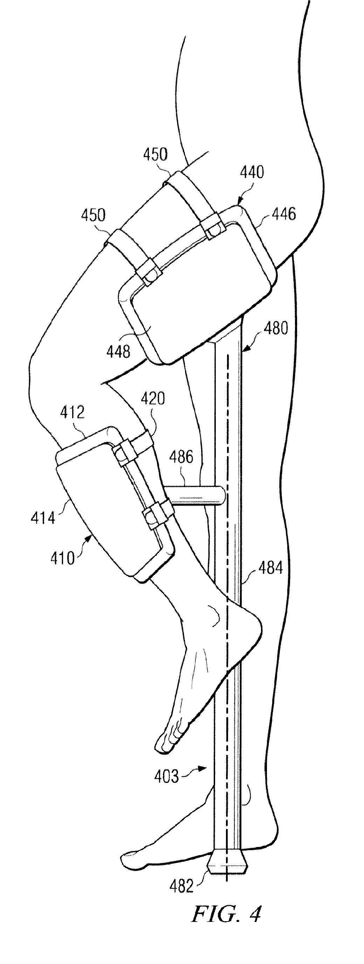

FIGURE 4 illustrates an example of a load redistribution system **403** for ambulation of a subject having a lower extremity impairment, in accordance with embodiments of the present disclosure. As shown in FIGURE 4, load redistribution system **403** may include lower cuff **410**, upper cuff **440**, and rod assembly **480**.

Lower cuff **410**, as shown, may include anterior pad **412**, anterior pad shell **414**, calf collar **420**, and tension adjustment fastener **426**. Lower cuff **410** may be secured on a subject's lower leg in a manner similar to cuffs **110** and **310**. Upper cuff **440**, as shown, may include, posterior pad **446**, posterior pad shell **448**, calf collar **450**, and tension adjustment fastener **456**. Upper cuff **440** may be secured on a subject's upper leg in a manner similar to cuffs **140** and **340**. Rod assembly **480**, as shown, may include foot **482**, vertical rod **484**, and lateral bar **486**.

Lateral bar **486** may be fixed to lower cuff **410** at one end and fixed to vertical rod **484** at the other as shown. In some embodiments, a lower cuff may be fixed directly to a vertical rod allowing the lateral bar to be omitted. Upper cuff **440** may be fixed directly to vertical rod **484**. In some embodiments, a lateral bar may be interposed between an upper cuff and a vertical rod. Rod assembly **480** may comprise one or more rigid materials capable of bearing a subject's weight. Examples of such materials include, without limitation, steel, a steel alloy, aluminum, an aluminum alloy, titanium, a titanium alloy, carbon fiber, wood, fiberglass and combinations thereof.

Variations in the weight of a subject, the positioning of the cuff(s), and/or the dimensions of the cuff(s) may influence the pressure on a subject's leg. In some embodiments, the pressure applied by a cuff on a subject's leg may be up to about 5 psi, up to about 7.5 psi, up to about 10 psi, up to about 15 psi, up to about 20 psi, up to about 25 psi, and/or up to about 30 psi. In some embodiments, a system may include one or more sensors (*e.g.*, pressure sensors). One or more sensors configured to detect the pressure at a particular location and/or the load born and/or or shifted

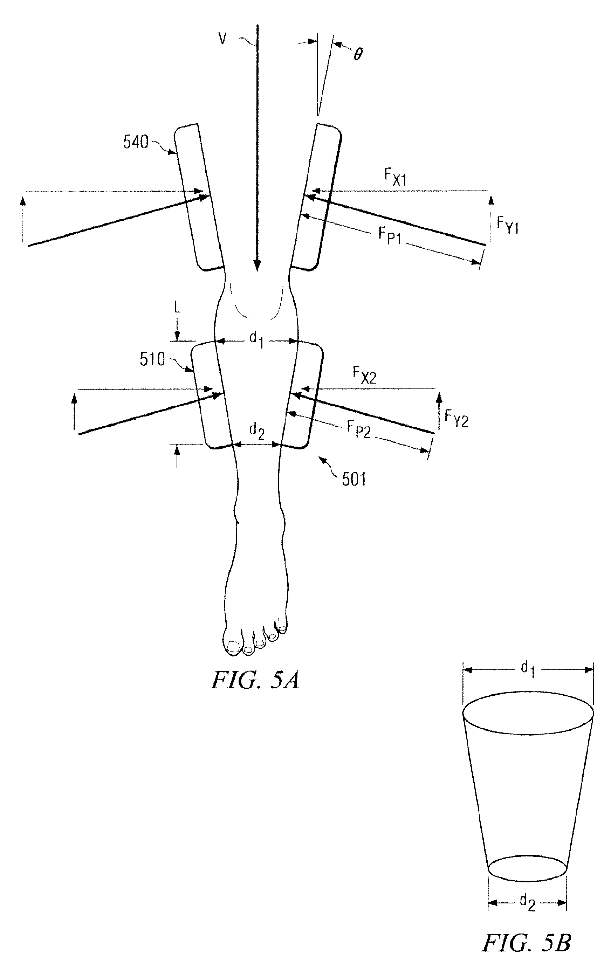

FIGURE 5A and 5B illustrate an example of a load redistribution system **501** for ambulation of a subject having a lower extremity impairment, in accordance with embodiments of the present disclosure. As shown in FIGURE 5A, load redistribution system **501** may support a subject's weight through lower strut **508** (not expressly shown), lower cuff **510** (comprising circumferential pad **511**), upper strut **538** (not expressly shown), and upper cuff **540** (comprising circumferential pad **541**). FIGURE 5A illustrates a method for calculating the pressure to be applied to a subject's leg as system **501** bears a subject's weight. Subject's weight is represented as vector V. As depicted, pads **511** and **541** are positioned at an angle θ relative to vector V. The force applied to pad **511** is represented as vector $F_{P1}$ and the force applied to pad **511** is represented as vector $F_{P2}$. The horizontal component of vector $F_{P1}$ is represented by vector $F_{X1}$ and the vertical component is represented by vector $F_{Y1}$. The horizontal component of vector $F_{P2}$ is represented by vector $F_{X2}$ and the vertical component is represented by vector $F_{Y2}$. Since system **501** is configured to bear a subject's full weight (w),

$$w = F_{Y1} + F_{Y2} \qquad \text{(Equation 1)}$$

If the subject's weight (w) is distributed evenly between pads **511** and **541**,

$$w/2 = F_{Y1} = F_{Y2} \qquad \text{(Equation 2)}$$

Thus, since

$$\sin(\theta) = F_{Y1} / F_{P1} \qquad \text{(Equation 3)}$$

the force $F_{P1}$ applied to a subject's leg may be calculated as follows:

$$F_{P1} = F_{Y1} / \sin(\theta) \qquad \text{(Equation 4)}$$

The area of cuff **510** is given by Equation 5:

$$\text{Area} = [(d_1 + d_2)/2] * L * \pi \qquad \text{(Equation 5)}$$

Thus, the pressure (P) on a subject's leg at cuff **510** is given by Equation 4:

$$P = \frac{F_{Y1} / \sin(\theta)}{[(d_1 + d_2)/2] * L * \pi} \qquad \text{(Equation 6)}$$

Therefore, if pad **511** is positioned at an angle of 7° ($\theta$), $d_1$ is 5.41 inches, $d_2$ is 3.5 inches, L is 7 inches, and the subject's weight is 180 pounds, the pressure (P) is given by

$$P = \frac{(w/2) / \sin(\theta)}{[(d_1 + d_2)/2] * L * \pi} \qquad \text{(Equation 7)}$$

$$P = \frac{(180/2) / \sin(7)}{[(5.41 + 3.5)/2] * 7 * \pi} \qquad \text{(Equation 8)}$$

$$P = 7.5 \text{ psi}$$

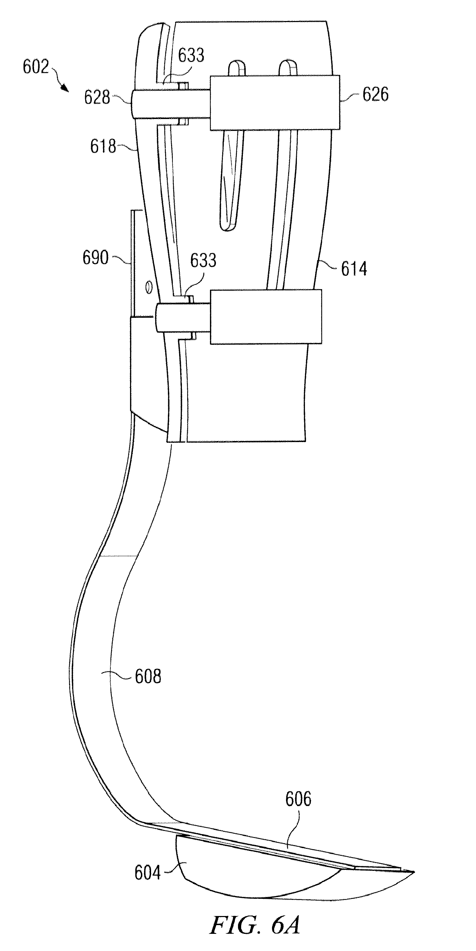

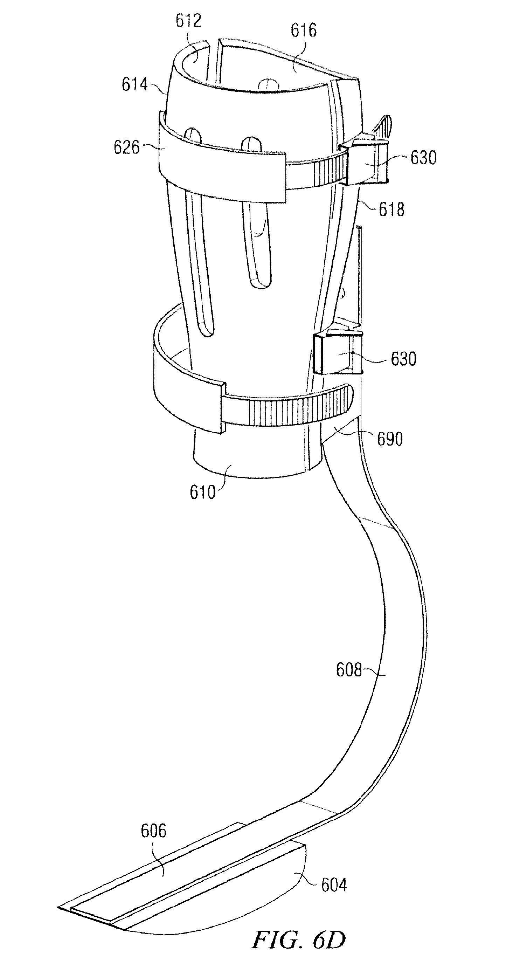

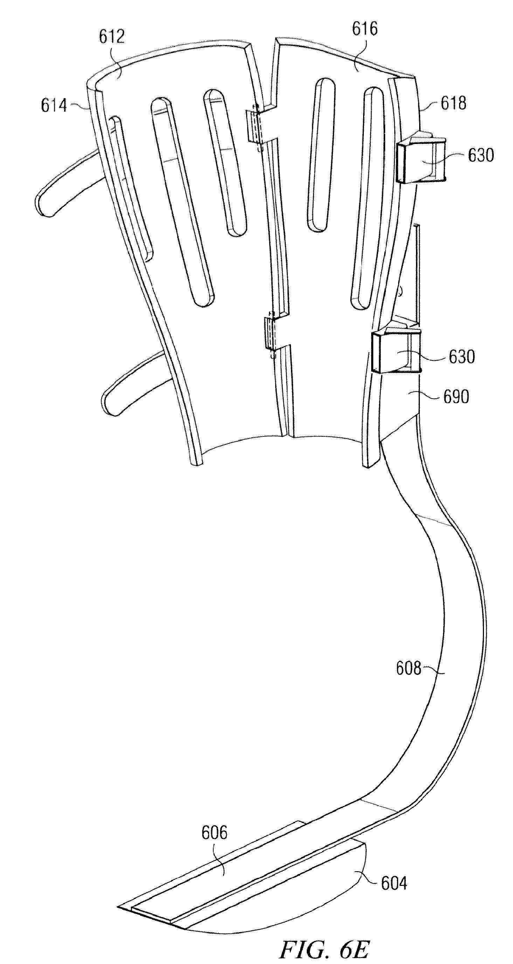

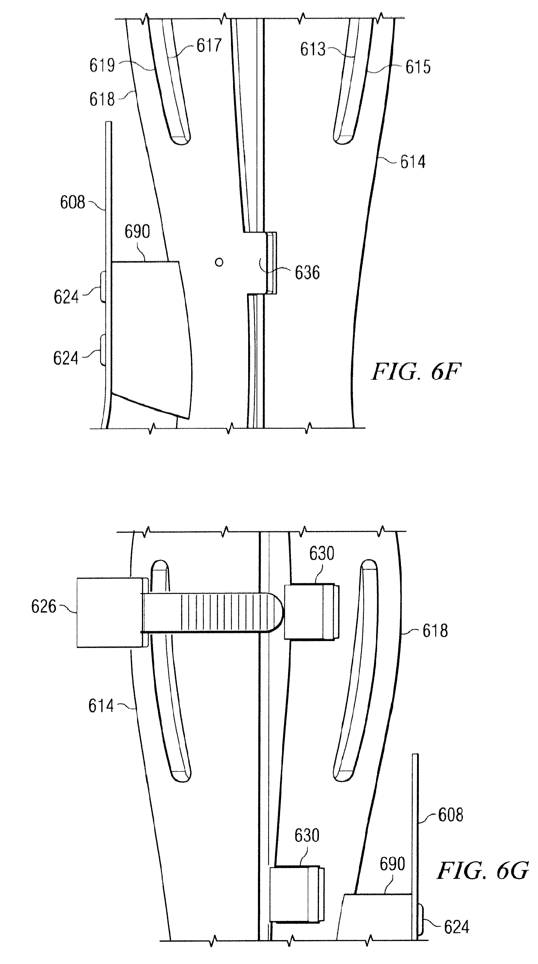

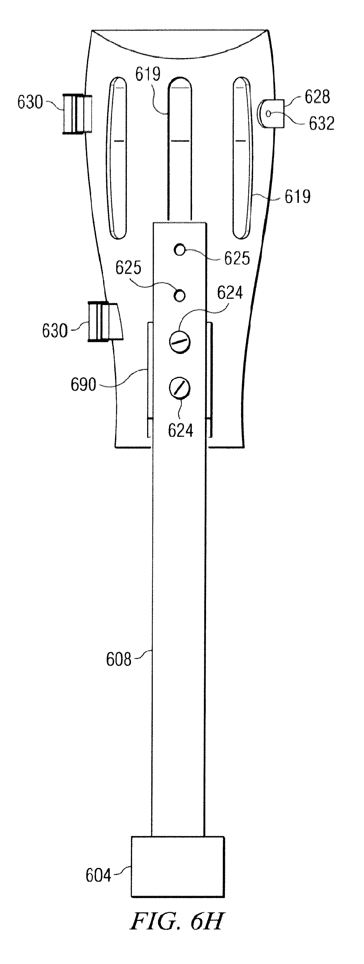

FIGURES 6A-6H illustrate an example of a load redistribution system **602** for ambulation of a subject having a lower extremity impairment, in accordance with embodiments of the present disclosure. As shown in FIGURES 6A-6H, load redistribution system **602** may include outsole **604**, platform **606**, strut **608**, support **690**, and cuff **610**.

Outsole **604** may comprise a foot pad, the bottom of which may be rounded (*e.g.*, rocker outsole) to facilitate walking. The bottom may be ridged as pictured or may have another tread pattern to aid ambulation. Outsole **604** may comprise a hard rubber or other suitable material. Outsole **604** may be generally rectangular in shape as depicted. Other regular and/or irregular shapes may be suitable and/or desirable in some embodiments. Platform **606** may be contiguous with strut **608** as shown or may be a separate piece fixedly attached to strut **608**. In some embodiments, outsole **604** and platform **606** may be adjustable (*e.g.,* fore and aft) relative to each other. The lower surface of platform **606** may be affixed to outsole) **604** with any type of fastener and/or adhesive. Platform **606** may sit atop outsole **604** as shown. In some embodiments, platform **606** may be recessed within outsole **604,** for example, so that the upper surface of outsole **604** is flush with the upper surface of platform **606**. Platform **606** may have any regular or irregular shape. Platform **606** may be somewhat smaller than outsole **604** as depicted or may be sized to match the size of outsole **604**. In some embodiments, it may be desirable for platform **606** to be larger than outsole **604**. Platform **606** may comprise a rigid material suitable for bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof.

System **602** may include strut **608** positioned substantially behind a subject's leg when the leg is secured in system **602.** At least a portion of strut **608** may be fixedly connected to and/or contiguous with a posterior portion of platform **606**. At least a portion of strut **608** extends from a posterior portion of platform **606** in the same plane as platform **606** or a plane generally parallel to the plane of platform **606** ("horizontal" portion). At least a portion of strut **608** rises above the plane of platform **606** and generally perpendicular to the plane of platform **606** ("vertical" portion). This vertical portion may have a lengthwise axis that is approximately parallel to the lengthwise axis of a subject's leg (*e.g.,* lower leg). Strut **608** may include bend **607** interposed between the horizontal and vertical portions of strut **608**. Bend **607** may be configured to bend or curve upward from platform **606** in a manner that permits a subject a normal or generally normal stride (*e.g.,* heel to toe contact with the ground). Strut **608** may comprise one or more rigid materials capable of bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof. While strut **608** may be rigid to support a subject's weight, some resilience/flexibility may be tolerated and/or desirable in some embodiments.

As depicted, cuff **610** may comprise anterior pad **612**. anterior pad shell **614**, posterior pad **616**, posterior pad shell **618,** and tension adjustment fastener **626**. Pad **612** may include one or more apertures **613**. Shell **614** may include one or more apertures **615**. Pad **616** may include one or more apertures **617**. Shell **618** may include one or more apertures **619**. Pad apertures **613** and **617** may be independently aligned (*e.g.*, partially or completely) with shell apertures **615** and **619,** respectively. Pads **612** and/or **616** may independently comprise flexible, elastomeric, and/or resilient materials including, for example, polyurethane, polyethylene, neoprene, ethylene vinyl acetate, foam, silicone, rubber, and the like. Pad shells **614** and/or **618** may independently comprise a moldable plastic. In some embodiments, pad shells **614** and/or **618** may independently comprise steel, a steel alloy, aluminum, an aluminum alloy, titanium, a titanium alloy, carbon fiber, an aramid fiber, a para-aramid fiber (*e.g.*, KEVLAR), fiberglass, and combinations thereof. Pad shells **614** and/or **618** comprising a moldable material may be custom fit to a subject. A moldable plastic may include, for example, a thermoplastic (*e.g.*, capable of more than one cycle of melting, molding, and setting) and/or a thermosetting plastic (*e.g.,* capable of one cycle of melting, molding, and setting). Pads **612** and/or **616** may be adhered, welded, bonded, stitched, or otherwise fixed to pad shells **614** and/or **618,** respectively.

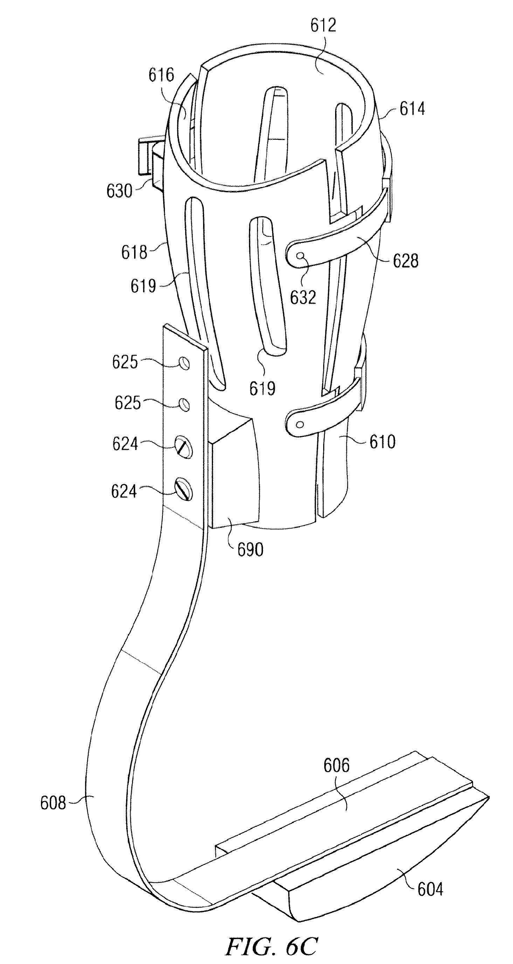

As shown in FIGURE 6C, pad shell **618** may be fixedly attached to support **690**. Pad shells **614** and **618** may be attached to each other through one or more hinges **633**. Support **690** may be attached to strut **608** - one or more screws **624** may be secured to support **690** through one or more holes **625** in strut **608**. As shown in FIGURE 6C, a plurality of holes **625** along a vertical axis of strut **608** affords a health care worker and/or a subject the opportunity to adjust the height of cuff **610**.

Tension adjustment fastener **626** may include strap **628**, cinch **630,** and anchor **632**. Strap **628** maybe fixed to posterior pad shell **618** via anchor **632**. Cinch **630** may be fixed to posterior pad shell **618** at a point along the circumference of shell **618** and spaced away from anchor **632**. Cinch **630** may be configured to receive and releasably grip strap **628**. In use, strap **628** may be threaded through cinch **630** and pulled tight to apply a desired amount of pressure on the subject's lower leg. Cinch **630** may then be closed to fix strap **628** in its position. System **602** may include one, two (pictured), three or more tension adjustment fasteners **626**.

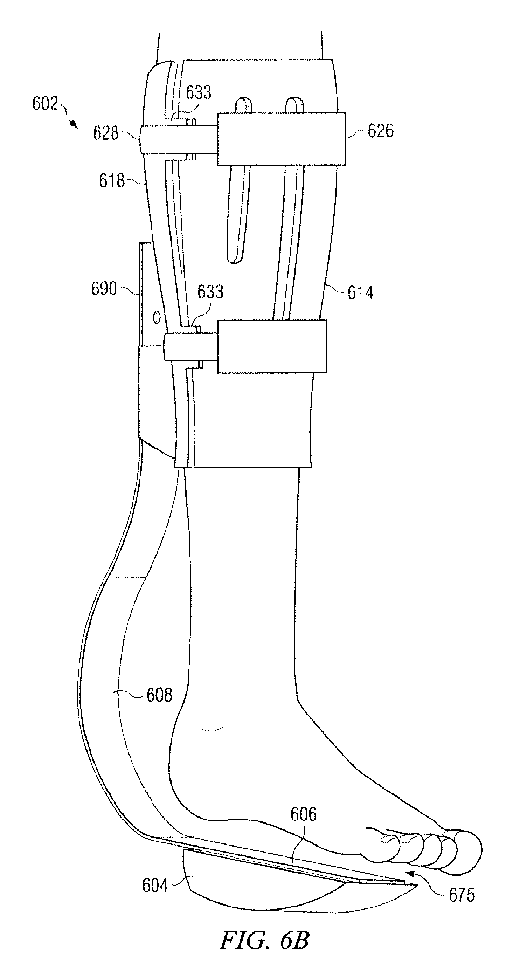

FIGURES 6B illustrates an example load redistribution system **602** for ambulation of a subject having a lower extremity impairment, in accordance with embodiments of the present disclosure. As shown in FIGURE 6B, system **602** may be configured (*e.g.*, formed and/or adjusted) to include gap **675** between a subject's foot **672** and the higher of outsole **604** and platform **606**. Gap **675** relieves subject's foot **672** and/or other lower extremities from bearing weight by diverting at least a portion of the load elsewhere (*e.g.*, all or substantially all of the load). In some embodiments, gap **675** may be any suitable distance that avoids (*e.g.*, minimizes, eliminates) loading foot **672** and/or other lower extremities. The extent of gap **675** may depend, in part, upon the size and weight of the subject user. For example, a subject with a long foot may be inclined and/or compelled to support some weight on foot **672** at some points in a walking cycle (*e.g.*, when foot **672** is at it's most rearward position with the toes nearest the ground and the heal elevated). It may be desirable in such situations to adjust gap **675** to be larger. A subject, for example, a smaller subject, may desire and/or require a smaller gap **675,** for example, to reduce or minimize the difference in length between the effected leg with system **602** and a healthy leg. In some embodiments, gap **675** may be from about 1 cm to about 5 cm, from about cm to about 15 cm, from about 1 cm to about 10 cm, from about 1 cm to about 20 cm, from about 2 cm to about 5 cm, from about 2 cm to about 10 cm, from about 2 cm to about 15 cm, and/or from about 2 cm to about 20 cm. Gap **675** may be less than about 1 cm or more than 20 cm in some embodiments.

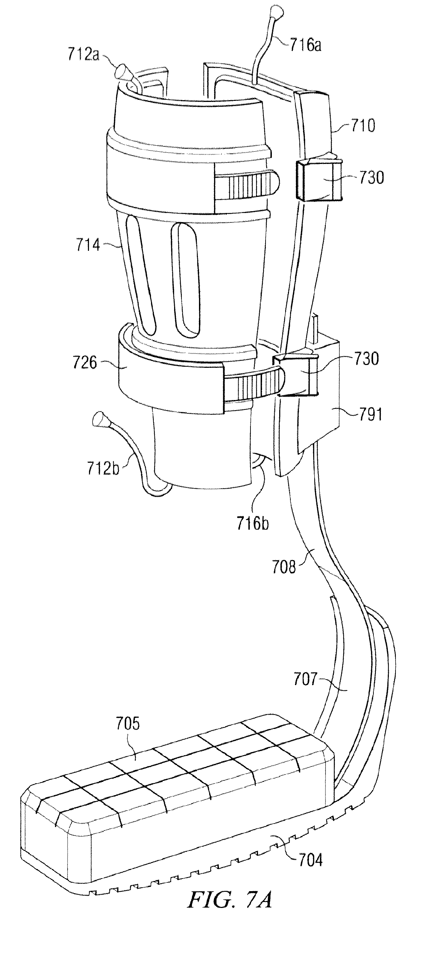

FIGURES 7A-7H illustrate an example of a load redistribution system **702** for ambulation of a subject having a lower extremity impairment, in accordance with embodiments of the present disclosure. As shown in FIGURES 7A-7H, load redistribution system **702** may include outsole **704**, pad **705**, platform **706**, strut **708**, support **791,** and cuff **710**.

Outsole **704** may comprise a foot pad, the bottom of which may be rounded (*e.g.*, rocker outsole) to facilitate walking. The bottom may be ridged as pictured or may have another tread pattern to aid ambulation. Outsole **704** may comprise a hard rubber or other suitable material. Outsole **704** may have any regular or irregular shape. For example, outsole **704** may be configured to resemble the tread of a running shoe, a dress shoe, and/or a boot. As depicted, outsole **704** may include a flange that extends from the plane of the walking surface in a generally vertical direction along the back of strut **708**. This flange may facilitate traction and/or a normal or improved stride. Other regular and/or irregular shapes may be suitable and/or desirable in some embodiments. Platform **706** may be contiguous with strut **708** as shown or may be a separate piece fixedly attached to strut **708**. In some embodiments, outsole **704** and platform **706** may be adjustable (*e.g.*, fore and aft) relative to each other. The lower surface of platform **706** may be affixed to outsole **704** with any type of fastener and/or adhesive. Platform **706** may sit atop outsole **704** as shown. In some embodiments, platform **706** may be recessed within outsole **704,** for example, so that the upper surface of outsole **704** is flush with the upper surface of platform **706**. Platform **706** may have any regular or irregular shape. Platform **706** may be somewhat smaller than outsole **704** as depicted or may be sized to match the size of outsole) **704**. In some embodiments, it may be desirable for platform **706** to be larger than outsole **704**. Platform **706** may comprise a rigid material suitable for bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof.

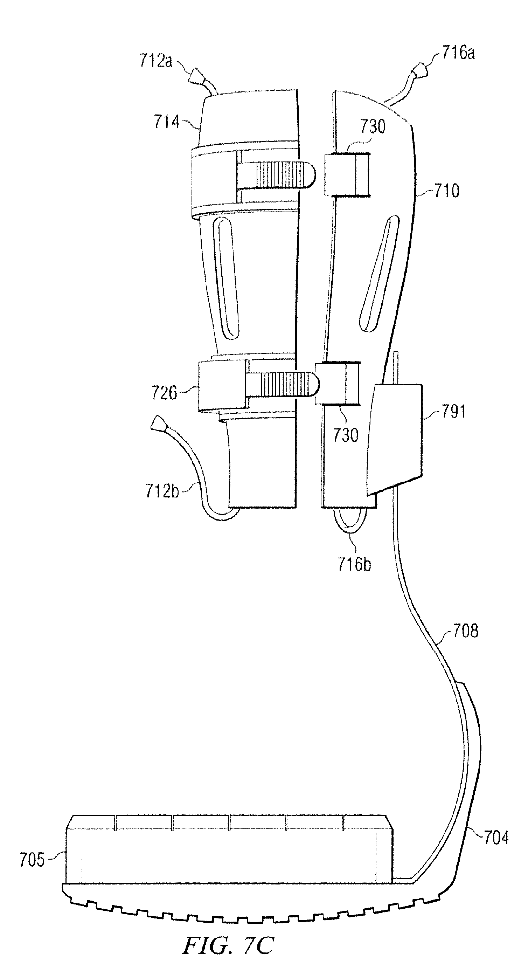

System **702** may include strut **708** positioned substantially behind a subject's leg when the leg is secured in system **702**. At least a portion of strut **708** may be fixedly connected to and/or contiguous with a posterior portion of platform **706**. Strut **708** may have an S-shaped profile as shown in Figure 7C or it may form and "L" with platform **706** (not expressly illustrated). At least a portion of strut **708** extends from a posterior portion of platform **706** in the same plane as platform **706** or a plane generally parallel to the plane of platform **706** ("horizontal" portion). At least a portion of strut **708** rises above the plane of platform **706** and generally perpendicular to the plane of platform **706** ("vertical" portion). This vertical portion may have a lengthwise axis that is approximately parallel to the lengthwise axis of a subject's leg (*e.g.*, lower leg). Strut **708** may include bend **707** interposed between the horizontal and vertical portions of strut **708**. Bend **707** may be configured to bend or curve upward from platform **706** in a manner that permits a subject a normal or generally normal stride *(e.g.,* heel to toe contact with the ground). Strut **708** may comprise one or more rigid materials capable of bearing a subject's weight. Examples of such materials include, without limitation, steel, a steel alloy, aluminum, an aluminum alloy, titanium, a titanium alloy, carbon fiber, wood, an aramid fiber, a para-aramid fiber (*e.g.*, KEVLAR), fiberglass, and combinations thereof. While strut **708** may he rigid to support a subject's weight, some resilience/flexibility may be tolerated and/or desirable in some embodiments.

As depicted, cuff **710** may comprise anterior bladder **712**, anterior shell **714**, posterior bladder **716,** posterior shell **718,** and tension adjustment fastener **726**. Bladder **712** may include one or more protrusions **713**. Shell **714** may include one or more apertures **715**. Bladder **716** may include one or more protrusions **717**. Shell **718** may include one or more apertures **719**. Bladder protrusions **713** and **717** may be aligned with shell apertures **715** and **719,** respectively to reduce or prevent, for example, movement of bladders relative to shells. Bladders **712** and/or **716** may independently comprise flexible, elastomeric, and/or resilient materials including, for example, polyurethane, polyethylene, neoprene, ethylene vinyl acetate, foam, silicone, rubber, and the like. Each bladder may have an inlet (**712a** and/or **716a**) and an outlet (**712b** and/or **716b**). System **702** may be custom fit to a subject by mounting system **702** on the subject and subsequently injecting a fill material into a bladder inlet (**712a** and/or **716a**). Fill material may be injected in some embodiment until it begins to be released from bladder outlet **(712b** and/or **716b).** In some embodiments, a fill material may be flowable (*e.g.*, a liquid) when it enters a bladder. A fill material may later convert and/or be converted to a resilient semi-resilient, or non-resilient gel or non-liquid (*e.g.*, solid) material. Examples of fill materials include, for example, foam, spray foam, gel foam, and/or combinations thereof.

Shells **714** and/or **718** may independently comprise a moldable plastic. In some embodiments, shells **714** and/or **718** may independently comprise steel, a steel alloy, aluminum, an aluminum alloy, titanium, a titanium alloy, carbon fiber, an aramid fiber, a para-aramid fiber (*e.g.*, KEVLAR), fiberglass, and combinations thereof. Shells **714** and/or **718** comprising a moldable material may be custom fit to a subject. A moldable plastic may include, for example, a thermoplastic (*e.g.*, capable of more than one cycle of melting, molding, and setting) and/or a thermosetting plastic (*e.g.*, capable of one cycle of melting, molding, and setting). Bladders **712** and/or **716** may be adhered, welded, bonded, stitched, or otherwise fixed to shells **714** and/or **718,** respectively.

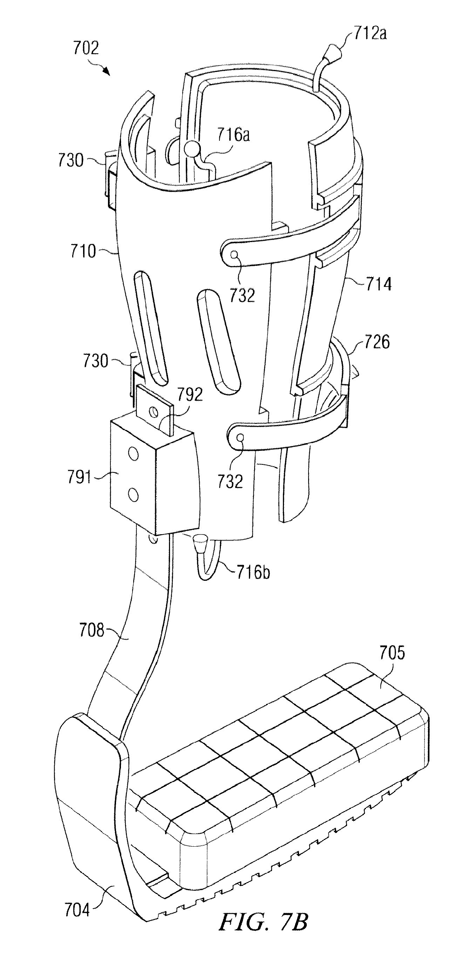

As shown in FIGURES 7B, shell **718** may be fixedly attached to support **791**. Support **791** may be attached to strut **708** - one or more screws **724** may be secured to support **791** through one or more holes **725** in strut **708**. As shown in FIGURE 7B, a plurality of holes **725** along a vertical axis of strut **708** affords a health care worker and/or a subject the opportunity to adjust the height of cuff **710**.

Tension adjustment fastener **726** may include strap **728,** cinch **730**, and anchor **732**. Strap **728** maybe fixed to posterior shell **718** via anchor **732**. Cinch **730** may be fixed to posterior shell **718** at a point along the circumference of shell **718** and spaced away from anchor **732**. Cinch **730** may be configured to receive and releasably grip strap **728**. In use, strap **728** may be threaded through cinch **730** and pulled tight to apply a desired amount of pressure on the subject's lower leg. Cinch **730** may then be closed to fix strap **728** in its position. System **702** may include one, two (pictured), three or more tension adjustment fasteners **726.**

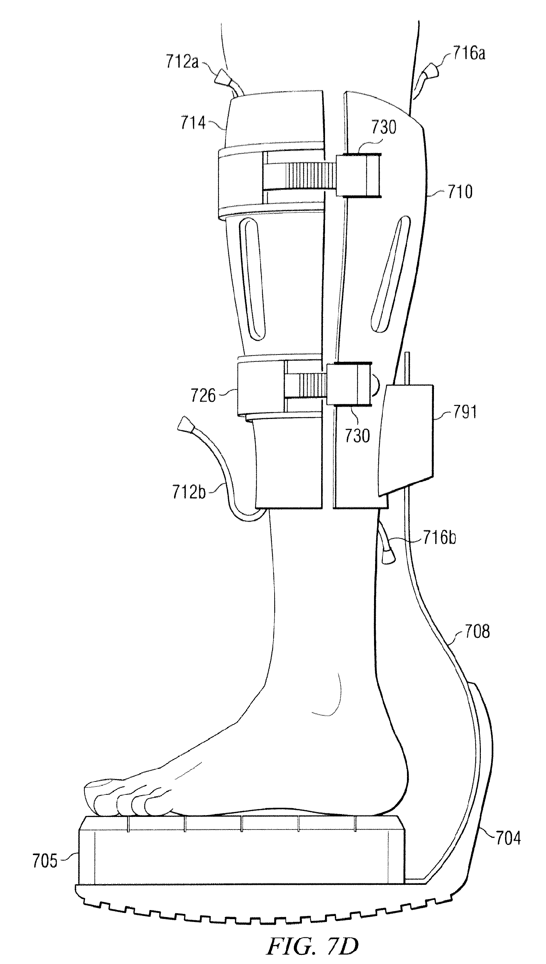

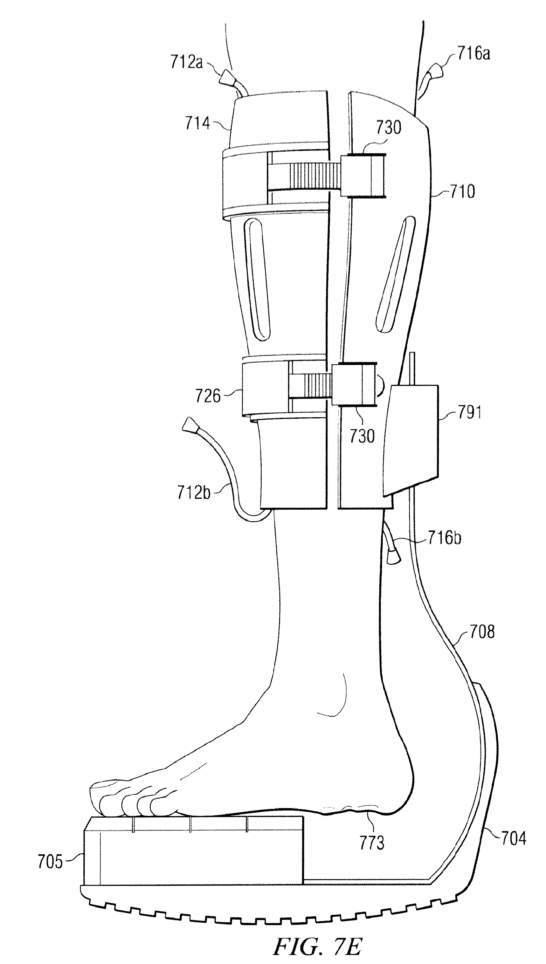

FIGURES 7D and 7E illustrate an example load redistribution system **702** for ambulation of a subject having a lower extremity impairment, in accordance with embodiments of the present disclosure. As shown in FIGURE 7D, system **702** may include pad **705** positioned to apply just enough pressure to the bottom of a subject's foot (*e.g.*, the toes and/or the ball of the foot) to hold it generally perpendicular to the long axis of the lower leg (*e.g.*, tibia and/or fibula). As shown in FIGURE 7E, one or more segments of pad **705** may be removed to accommodate the particular desires or needs of a subject with ulcer **773** or other foot injury. Pad **705** may span up to the entire gap **775**. Pad **705** may comprise flexible, elastomeric, and/or resilient materials including, for example, polyurethane, polyethylene, neoprene, ethylene vinyl acetate, foam, silicone, rubber, and the like.

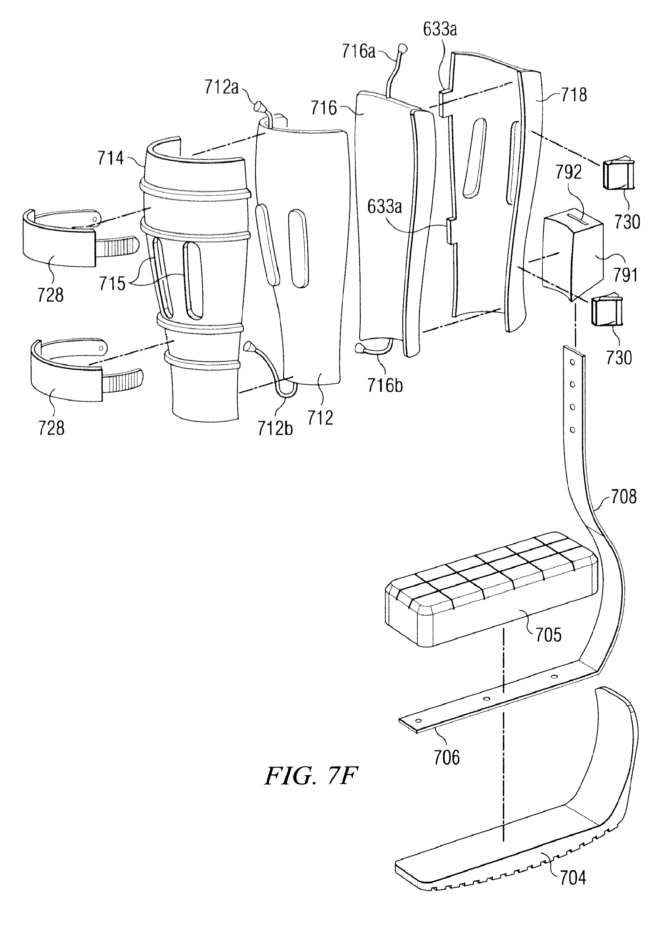

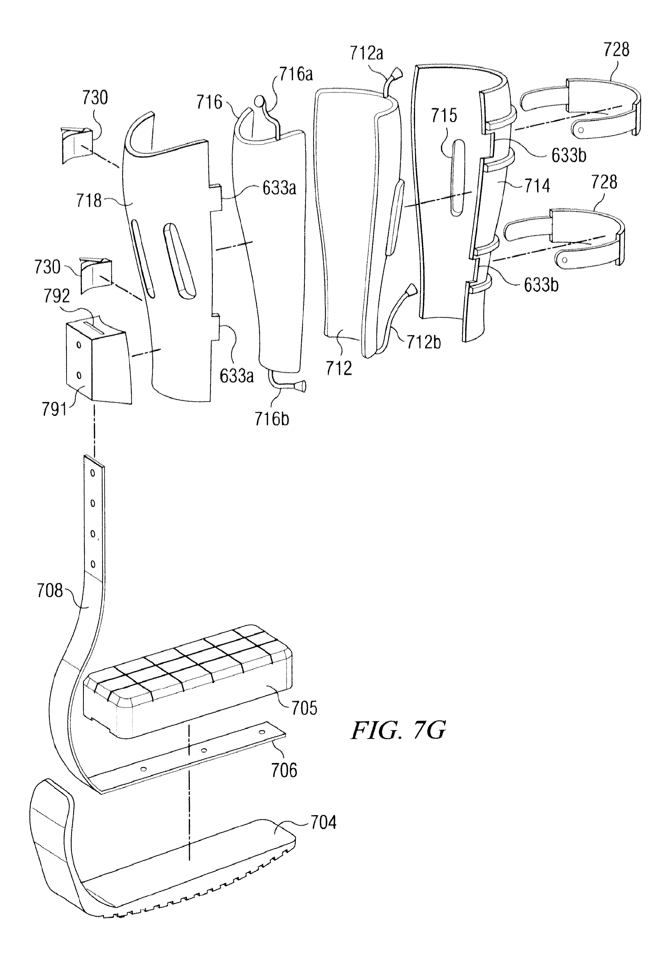

FIGURES 7F and 7G illustrate exploded views of an example load redistribution system **702** for ambulation of a subject having a lower extremity impairment, in accordance with embodiments of the present disclosure. As shown in FIGURE 7G, pad shell **718** may include protrusions **733a** that complement slots **733b** in pad shell **714**. When pad shells **714** and **718** come together (*e.g.*, around a subject's leg), protrusions **733a** fit into corresponding slots **733b** and may stabilize the pad shell sections relative to each other.

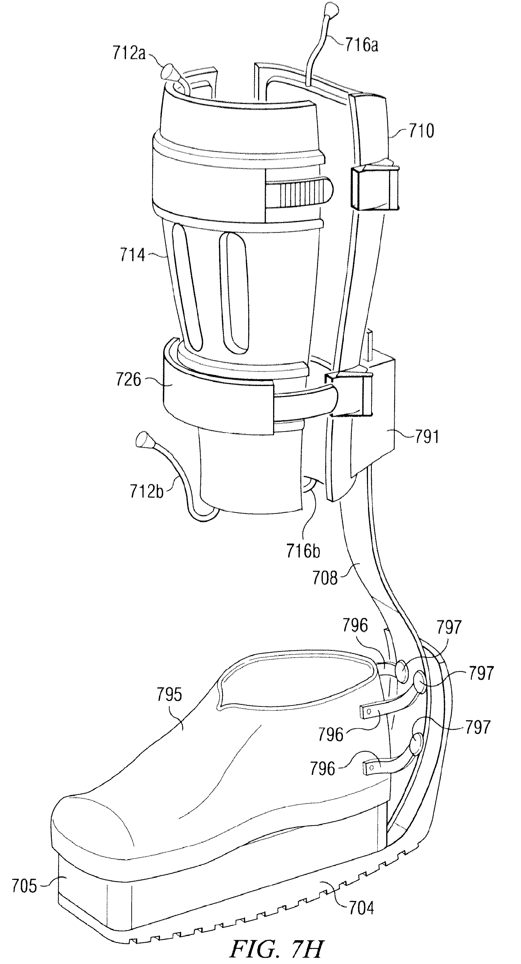

FIGURE 7H illustrates an example load redistribution system **702** for ambulation of a subject having a lower extremity impairment, in accordance with embodiments of the present disclosure. As shown in FIGURE 7H, system **702** may include shell **795** configure to shield and/or protect a subject's foot from incidental contact with other objects. Shell **795** may be secured to strut **708** with straps **796** and snaps **797** as illustrated. Additional straps, snaps, and/or other fasteners may be added to secure shell **795** (*e.g.*, between shell **795** and outsole **704** and/or platform **708.**

As will be understood by those skilled in the art who have the benefit of the instant disclosure, other equivalent or alternative compositions, devices, methods, and systems for redistributing a load can be envisioned without departing from the description contained herein. Accordingly, the manner of carrying out the disclosure as shown and described is to be construed as illustrative only.

Persons skilled in the art may make various changes in the shape, size, number, and/or arrangement of parts without departing from the scope of the instant disclosure. For example, the position and number of struts, pads, collars, and/or cuffs may be varied. In some embodiments, pads, pad shells, collars, and/or cuffs may be interchangeable. Interchangeability may allow the pressure exerted on a lateral surface of a subject's leg to be custom adjusted (*e.g.*, by substituting larger or smaller pads). In addition, the size of a device and/or system may be scaled up (*e.g.,* to be used for adult subjects) or down (*e.g.*, to be used for juvenile subjects) to suit the needs and/or desires of a practitioner. Also, where ranges have been provided, the disclosed endpoints may be treated as exact and/or approximations as desired or demanded by the particular embodiment. In addition, it may be desirable in some embodiments to mix and match range endpoints. All or a portion of a device and/or system for redistributing a load may be configured and arranged to be disposable, serviceable, interchangeable, and/or replaceable. These equivalents and alternatives along with obvious changes and modifications are intended to be included within the scope of the present disclosure. Accordingly, the foregoing disclosure is intended to be illustrative, but not limiting, of the scope of the disclosure as illustrated by the following claims.

The following clauses, which are not claims, describe various aspects of the present disclosure:

Clause 1. A system for redistributing weight away from a subject's foot, the system comprising: a platform; at least one vertical support fixed to the platform and extending upwardly from the platform; and at least one cuff (a) configured to surround and releasably grip at least a portion of the subject's leg other than the foot and (b) mounted to the at least one vertical support at a vertical position along the at least one vertical support sufficient to suspend the subject's foot in a non-weight-bearing position above the platform during ambulation, wherein the platform, the at least one vertical support, and the at least one cuff together are configured to bear at least the subject's full weight.

2. A system for redistributing weight away from a subject's foot according to Clause 1, wherein the at least one vertical support comprises a strut positioned relative to the subject's leg on which the system is worn opposite the subjects sagittal plane or opposite the subject's coronal plane.

3. A system for redistributing weight away from a subject's foot according to Clause 1, wherein the at least one vertical support comprises a strut positioned generally behind the subjects leg on which the system is worn.

4. A system for redistributing weight away from a subject's foot according to Clause 3, wherein the strut has an S-curve profile.

5. A system for redistributing weight away from a subject's foot according to Clause 1, wherein the at least one vertical support comprises a strut positioned generally outside the subject's leg on which the system is worn

6. A system for redistributing weight away from a subject's foot according to Clause 1, wherein the at least one vertical support extends distal to the subject's foot.

7. A system for redistributing weight away from a subject's foot according to Clause 1, wherein the at least one cuff comprises an anterior shell, an opposing posterior shell, and at least one tension adjustment fastener configured to releasably connect the anterior shell and the posterior shell, wherein the anterior shell and the posterior shell are configured to combine to surround the circumference of the subject's leg.

8. A system for redistributing weight away from a subject's foot according to Clause 7, wherein the anterior shell comprises at least one fastener guide positioned across the anterior surface of the anterior shell and configured to receive at least a portion of the at least one tension adjustment fastener.

9. A system for redistributing weight away from a subject's foot according to Clause 7, wherein the anterior shell and/or the posterior shell comprise a moldable plastic selected from the group consisting of a thermoplastic, a thermosetting plastic, and combinations thereof.

10. A system for redistributing weight away from a subject's foot according to Clause 7, wherein the anterior shell or the posterior shell is adjustably mounted to the at least one vertical support.

11. A system for redistributing weight away from a subject's foot according to Clause 7, wherein the at least one cuff further comprises at least one pad fixed to the anterior shell and at least one pad fixed to the posterior shell.

12. A system for redistributing weight away from a subject's foot according to Clause 11 , wherein the pads independently comprise a material selected from the group consisting of polyurethane. polyethylene, neoprene, ethylene vinyl acetate, foam, silicone, rubber, and combinations thereof.

13. A system for redistributing weight away from a subject's foot according to Clause 7, wherein the at least one cuff further comprises at least one bladder fixed to the anterior shell and at least one bladder fixed to the posterior shell.

14. A system for redistributing weight away from a subject's foot according to Clause 13, wherein at least one of the bladders is at least partially filled with a fill material.

15. A system for redistributing weight away from a subject's foot according to Clause 14, wherein the fill material comprises a foam.

16. A system for redistributing weight away from a subject's foot according to Clause 1, wherein the cuff is configured to extend from about the gastroenemius/solius muscles to about the tibial plateau.

17. A system for redistributing weight away from a subject's foot according to Clause 1 further comprising an outsole fixed to the platform and configured to contact the ground during ambulation.

18. A system for redistributing weight away from a subject's foot according to Clause 17, wherein the outsole comprises a flange extending vertically along at least a portion of the back of the vertical support.

19. A system for redistributing weight away from a subject's foot according to Clause 1 further comprising a foot pad fixed to the platform and configured to contact at least a portion of the subject's foot and to hold it approximately perpendicular to the longitudinal axis of the subject's lower leg.

20. A system for redistributing weight away from a subject's foot according to Clause 19 further comprising a lateral bar interposed between the at least one cuff and the vertical rod.

21. A system for redistributing weight away from a subject's foot according to Clause 19 further comprising a second cuff, the second cuff (a) configured to surround and releasably grip at least a second portion of a subjects leg other than the foot and (b) mounted to the at least one vertical support at a vertical position along the at least one vertical support sufficient to suspend a subject's foot in a non-weight-bearing position above the platform during ambulation.

22. A system for redistributing weight away from a subject's foot according to Clause 19, wherein the vertical rod extends distal to the subject's foot.

23. A system for redistributing weight away from a subject's foot according to Clause 1 further comprising: a hinge attached to the upper end of the vertical support: a second vertical support rotatably attached to the hinge; and a second cuff, the second cuff (a) configured to surround and releasably grip at least a second portion of a subject's leg other than the foot and (b) mounted to the second vertical support at a vertical position along the second vertical support sufficient to cooperate with the first cuff to suspend a subject's foot in a non-weight-bearing position above the platform during ambulation.

24. A system for redistributing weight away from a subject's foot according to Clause 1 further comprising a mated shoe comprising a lift configured to vertically align the suspended foot and the other foot.

Clause 25. A method for facilitating ambulation of a subject having a leg with an impaired lower extremity, the method comprising: suspending at least a portion of the impaired lower extremity in a non-weight bearing position using a load redistribution system; and redistributing the weight to one or more unimpaired regions of the leg using the load redistribution system, the load redistribution system comprising: a platform; at least one vertical support fixed to the platform and extending upwardly from the platform; and at least one cuff mounted to the at least one strut at a position sufficient to suspend the at least a portion of the impaired lower extremity in a non-weight-bearing position above the platform during ambulation, wherein the platform, the at least one strut, and the at least one cuff are configured to (a) bear at least the subject's full weight and (b) distribute the weight born to at least a portion of the subject's leg other than the subject's foot.

26. A method for facilitating ambulation of a subject according to Clause 25, wherein the at least one vertical support comprises a strut positioned relative to the subjects leg on which the system is worn opposite the subject's sagittal plane or opposite the subject's coronal plane.

27. A method for facilitating ambulation of a subject according to Clause 25, wherein the at least one vertical support comprises a strut positioned generally behind the subject's leg on which the system is worn.

28. A method for facilitating ambulation of a subject according to Clause 25, wherein the strut has an S-curve profile.

29. A method for facilitating ambulation of a subject according to Clause 25, wherein the at least one vertical support comprises a strut positioned generally outside the subject's leg on which the system is worn

30. A method for facilitating ambulation of a subject according to Clause 25, wherein the at least one vertical support extends distal to the subject's foot.

31. A method for facilitating ambulation of a subject according to Clause 25, wherein the at least one cuff comprises an anterior shell, an opposing posterior shell, and at least one tension adjustment fastener configured to releasably connect the anterior shell and the posterior shell, wherein the anterior shell and the posterior shell are configured to combine to surround the circumference of the subject's leg.

32. A method for facilitating ambulation of a subject according to Clause 31, wherein the anterior shell comprises at least one fastener guide positioned across the anterior surface of the anterior shell and configured to receive at least a portion of the at least one tension adjustment fastener.

33. A method for facilitating ambulation of a subject according to Clause 31, wherein the anterior shell and/or the posterior shell comprise a moldable plastic selected from the group consisting of a thermoplastic, a thermosetting plastic, and combinations thereof.

34. A method for facilitating ambulation of a subject according to Clause 31, wherein the anterior shell or the posterior shell is adjustably mounted to the at least one vertical support.

35. A method for facilitating ambulation of a subject according to Clause 31, wherein the at least one cuff further comprises at least one pad fixed to the anterior shell and at least one pad fixed to the posterior shell.

36. A method for facilitating ambulation of a subject according to Clause 35, wherein the pads independently comprise a material selected from the group consisting of polyurethane, polyethylene, neoprene, ethylene vinyl acetate, foam, silicone, rubber, and combinations thereof.

37. A method for facilitating ambulation of a subject according to Clause 31, wherein the at least one cuff further comprises at least one bladder fixed to the anterior shell and at least one bladder fixed to the posterior shell.

38. A method for facilitating ambulation of a subject according to Clause 37, wherein at least one of the bladders is at least partially filled with a fill material.

39. A method for facilitating ambulation of a subject according to Clause 38, wherein the fill material comprises a foam.

40. A method for facilitating ambulation of a subject according to Clause 25, wherein redistributing the weight to one or more unimpaired regions of the leg comprises redistributing the weight to one or more lateral surfaces of the leg.

41. A method for facilitating ambulation of a subject according to Clause 40, wherein redistributing the weight to one or more lateral surfaces of the leg further comprises adjusting the pressure applied to at least one lateral surface.

Clause 42. A load redistribution system for redistributing weight away from a subject's foot, the system comprising: a means for suspending a subject's foot in a non-weight-bearing position; and a means for redistributing a subject's weight to at least one surface on the subject's leg.

## Claims

1. A system for redistributing weight away from a subject's foot, the system comprising:

   a platform;
   at least one vertical support fixed to the platform and extending upwardly from the platform; and
   at least one cuff (a) configured to surround and releasably grip at least a portion of the subject's leg other than the foot and (b) mounted to the at least one vertical support at a vertical position along the at least one vertical support sufficient to suspend the subject's foot in a non-weight-bearing position above the platform during ambulation,

   wherein the platform, the at least one vertical support, and the at least one cuff together are configured to bear at least the subject's full weight; and
   wherein the at least one vertical support extends distal to the subject's foot.

2. A system for redistributing weight away from a subject's foot according to Claim 1,
   wherein the at least one cuff comprises an anterior shell, an opposing posterior shell, and at least one tension adjustment fastener configured to releasably connect the anterior shell and the posterior shell, wherein the anterior shell and the posterior shell are configured to combine to surround the circumference of the subject's leg, and wherein the anterior shell comprises at least one fastener guide positioned across the anterior surface of the anterior shell and configured to receive at least a portion of the at least one tension adjustment fastener.

3. A system for redistributing weight away from a subject's foot according to Claim 3, wherein the at least one cuff further comprises at least one bladder fixed to the anterior shell and at least one bladder fixed to the posterior shell.

4. A system for redistributing weight away from a subject's foot according to Claim 3, wherein at least one of the bladders is at least partially filled with a fill material.

5. A system for redistributing weight away from a subject's foot according to Claim 4, wherein the fill material comprises a foam.

6. A system for redistributing weight away from a subject's foot according to Claim 1, wherein the cuff is configured to extend from about the gastrocnemius/soleus muscles to about the tibial plateau.

7. A system for redistributing weight away from a subject's foot according to Claim 1 further comprising an outsole fixed to the platform and configured to contact the ground during ambulation, wherein the outsole comprises a flange extending vertically along at least a portion of the back of the vertical support.

8. A system for redistributing weight away from a subject's foot according to Claim 1 further comprising a foot pad fixed to the platform and configured to contact at least a portion of the subject's foot and to hold it approximately perpendicular to the longitudinal axis of the subject's lower leg; and further comprising a lateral bar interposed between the at least one cuff and the vertical support.

9. A system for redistributing weight away from a subject's foot according to Claim 1, wherein the single vertical support comprises a strut positioned generally behind the subject's leg on which the system is worn.

10. A system for redistributing weight away from a subject's foot according to Claim 9, wherein the strut has an S-curve profile.

11. A method for facilitating ambulation of a subject having a leg with an impaired lower extremity, the method comprising:

suspending at least a portion of the impaired lower extremity in a non-weight bearing position using a load redistribution system; and
redistributing the weight to one or more unimpaired regions of the leg using the load redistribution system, the load redistribution system comprising:

a platform;
at least one vertical support fixed to the platform and extending upwardly from the platform; and
at least one cuff mounted to the at least one strut at a position sufficient to suspend the at least a portion of the impaired lower extremity in a non-weight-bearing position above the platform during ambulation, wherein the platform, the at least one strut, and the at least one cuff are configured to (a) bear at least the subject's full weight and (b) distribute the weight born to at least a portion of the subject's leg other than the subject's foot,

wherein the at least one vertical support comprises a strut positioned generally outside the subject's leg on which the system is worn.

12. A method for facilitating ambulation of a subject according to Claim 11, wherein the at least one vertical support extends distal to the subject's foot.

13. A method for facilitating ambulation of a subject according to Claim 11, wherein the at least one cuff comprises an anterior shell, an opposing posterior shell, and at least one tension adjustment fastener configured to releasably connect the anterior shell and the posterior shell, wherein the anterior shell and the posterior shell are configured to combine to surround the circumference of the subject's leg, and wherein the anterior shell comprises at least one fastener guide positioned across the anterior surface of the anterior shell and configured to receive at least a portion of the at least one tension adjustment fastener.

14. A method for facilitating ambulation of a subject according to Claim 13, wherein the at least one cuff further comprises at least one bladder fixed to the anterior shell and at least one bladder fixed to the posterior shell.

15. A load redistribution system for redistributing weight away from a subject's foot, the system comprising:

a means for suspending a subject's foot in a non-weight-bearing position; and
a means for redistributing a subject's weight to at least one surface on the subject's leg.

*FIG. 1A*

*FIG. 1B*

FIG. 1C

FIG. 1D

FIG. 2A

FIG. 2B

FIG. 3A

FIG. 3B

316    312

318    314

FIG. 3C

310

FIG. 3D

FIG. 4

FIG. 5A

FIG. 5B

*FIG. 6A*

*FIG. 6B*

*FIG. 6C*

FIG. 6D

*FIG. 6E*

*FIG. 6F*

*FIG. 6G*

*FIG. 6H*

*FIG. 7A*

FIG. 7B

*FIG. 7C*

*FIG. 7D*

FIG. 7E

*FIG. 7F*

FIG. 7G

FIG. 7H

**Europäisches Patentamt**

**European Patent Office**

**Office européen des brevets**

# EUROPEAN SEARCH REPORT

Application Number

EP 16 18 9787

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/054962 A1 (BRADSHAW JASON L [US]) 10 March 2005 (2005-03-10) * abstract; figures 1,13,15 * * paragraphs [0002], [0015], [0033], [0034] * | 1-8, 11-15 | INV. A61F5/01 |
| X | US 6 997 891 B1 (VECSEY BRETT [US]) 14 February 2006 (2006-02-14) * abstract; figures 1-3 * * column 1, line 33 - column 3, line 37 * | 1-6, 11-15 | |
| X | FR 2 432 306 A (OETIKER HANS [CH]) 29 February 1980 (1980-02-29) * figures 1-5 * * page 1, line 36 - page 2, line 9 * * page 4, line 1 - page 6, line 31 * | 1-6, 11-15 | |
| X | US 2003/216675 A1 (ROONEY JOHN E [US]) 20 November 2003 (2003-11-20) * abstract; figure 1 * * paragraphs [0036] - [0049] * | 1-8,15 9,10 | |
| Y | | | **TECHNICAL FIELDS SEARCHED (IPC)** |
| Y | DE 199 05 544 A1 (BOECKH THOMAS [DE]; GUENTHER NORBERT [DE]; GOTTINGER FERDINAND [DE]) 17 August 2000 (2000-08-17) * column 3, lines 57-64; figures 3-4 * | 9,10 | A61F |

The present search report has been drawn up for all claims

| Place of search | Date of completion of the search | Examiner |
|---|---|---|
| Munich | 9 December 2016 | Dennler, Samuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 18 9787

This annex lists the patent family members relating to the patent documents cited in  the above-mentioned European search report.
The members are as contained in the European Patent Office EDP file on
The European Patent Office is in no way liable for these particulars which are merely given for the purpose of information.

09-12-2016

| Patent document cited in search report | | Publication date | Patent family member(s) | | Publication date |
|---|---|---|---|---|---|
| US 2005054962 | A1 | 10-03-2005 | US 2005054962 A1<br>US 2006116617 A1 | | 10-03-2005<br>01-06-2006 |
| US 6997891 | B1 | 14-02-2006 | NONE | | |
| FR 2432306 | A | 29-02-1980 | DE 2833482 A1<br>FR 2432306 A1 | | 14-02-1980<br>29-02-1980 |
| US 2003216675 | A1 | 20-11-2003 | NONE | | |
| DE 19905544 | A1 | 17-08-2000 | DE 19905544 A1<br>DE 19964578 B4<br>DE 29908981 U1 | | 17-08-2000<br>13-11-2008<br>16-12-1999 |

EPO FORM P0459

For more details about this annex : see Official Journal of the European Patent Office, No. 12/82